# EUROPEAN PATENT SPECIFICATION

(11) **EP 3 996 758 B1**
(45) Date of publication and mention of the grant of the patent: **26.07.2023**
(21) Application number: 20736383.9
(22) Date of filing: 09.07.2020
(51) Int. Cl.: A61L 15/22, A61L 15/32, A61L 15/42, A61L 15/64, A61L 24/00, A61L 24/04, A61L 24/10

(54) **HAEMOSTATIC POWDER**
HÄMOSTATISCHES PULVER
POUDRE HÉMOSTATIQUE

(30) Priority: 12.07.2019 EP 19186028
(43) Date of publication of application: 18.05.2022
(60) Divisional application: 23169050.4
(73) Proprietor: Cilag GmbH International, 6300 Zug (CH)
(72) Inventor: KEEREWEER, Abraham Reinier, 6534 AT Nijmegen (NL); FÉLIX LANAO, Rosa Pilar, 6534 AT Nijmegen (NL); OPSTEEN, Joost, 6534 AT Nijmegen (NL); BENDER, Johannes Caspar Mathias Elizabeth, 6534 AT Nijmegen (NL)
(74) Representative: Nederlandsch Octrooibureau
(86) International application number: PCT/EP2020/069442
(87) International publication number: WO 2021/009014

(56) References cited:
- WO-A1-2016/056901
- WO-A2-2010/059280
- WO-A2-2013/053759
- MARCEL A. BOERMAN ET AL: "Next Generation Hemostatic Materials Based on NHS-Ester Functionalized Poly(2-oxazoline)s", BIOMACROMOLECULES, vol. 18, no. 8, 25 July 2017 (2017-07-25), pages 2529-2538, XP055655362, US ISSN: 1525-7797, DOI: 10.1021/acs.biomac.7b00683

## Description

### TECHNICAL FIELD OF THE INVENTION

The present invention relates to a haemostatic powder that comprises at least 10 wt.% of particle agglomerates comprising:
- electrophilic polyoxazoline particles containing electrophilic polyoxazoline carrying at least 3 reactive electrophilic groups that are capable of reacting with amine groups in blood under the formation of a covalent bond; and
- nucleophilic polymer particles containing a nucleophilic polymer carrying at least 3 reactive nucleophilic groups that, in the presence of water, are capable of reacting with the reactive electrophilic groups of the electrophilic polyoxazoline under the formation of a covalent bond between the electrophilic polyoxazoline and the nucleophilic polymer.

When the haemostatic powder of the present invention is applied to a bleeding site, the particle agglomerates rapidly form a gel while at the same time binding to proteins present in the blood and in the surrounding tissue, thereby speeding up haemostasis.

### BACKGROUND OF THE INVENTION

Haemostasis is a tightly regulated process that maintains the blood flow through the vasculature simultaneously as a thrombotic response to tissue damage occurs. Maintaining haemostasis requires a complex interaction of the vessel wall, platelets, and the coagulation and fibrinolytic systems. There are two main phases of haemostasis: primary (ie, the cellular phase) and secondary (ie, the humoral phase).

Primary haemostasis begins immediately after endothelial disruption and is characterized by vasoconstriction, platelet adhesion, and formation of a soft aggregate plug. After the injury occurs, there is a temporary local contraction of vascular smooth muscle and the blood flow slows, promoting platelet adhesion and activation. Within 20 seconds of the injury, circulating von Willebrand factor attaches to the subendothelium at the site of injury and adheres to the glycoproteins on the surface of platelets. As platelets adhere to the injured surface, they are activated by contact with collagen-exposing receptors that bind circulating fibrinogen. A soft plug of aggregated platelets and fibrinogen is formed. This phase of haemostasis is short lived, and the soft plug can easily be sheared from the injured surface.

The soft platelet plug is stabilized during secondary haemostasis to form a clot. Vasoconstriction and the resultant reduction in blood flow are maintained by platelet secretion of serotonin, prostaglandin, and thromboxane while the coagulation cascade is initiated. The coagulation cascade is a series of dependent reactions involving several plasma proteins, calcium ions, and blood platelets that lead to the conversion of fibrinogen to fibrin. Coagulation factors are produced by the liver and circulate in an inactive form until the coagulation cascade is initiated. Then each step of the cascade is initiated and completed via a series of sequential and dependent coagulation factor activation reactions. In the final steps, thrombin converts the soluble plasma protein fibrinogen to the insoluble protein fibrin, while simultaneously converting factor XIII to factor Xllla. This factor conversion stabilizes the fibrin and results in cross-linking of the fibrin monomers, producing a stable clot.

During surgery, it is important to maintain a fine balance between bleeding and clotting, such that blood continues to flow to the tissues at the surgical site without excessive loss of blood, to optimize surgical success and patient outcome. Continuous bleeding from diffuse minor capillaries or small venules during surgery can obscure the surgical field, prolong operating time, increase the risk of physiologic complications, and expose the patient to risks associated with blood transfusion

Surgeons have an array of options to control bleeding, including mechanical and thermal techniques and devices as well as pharmacotherapies and topical agents.

One of the earliest topical haemostatic agents was cotton, in the form of gauze sponges. Although such materials concentrate blood and coagulation products via physical adsorption, they are not absorbed by the body, and upon removal, the clot may be dislodged, leading to further bleeding. Absorbable topical haemostatic agents have since been developed and provide useful adjunctive therapy when conventional methods of haemostasis are ineffective or impractical. Topical haemostatic agents can be applied directly to the bleeding site and may prevent continuous unrelenting bleeding. Haemostasis using topical agents also can avoid the adverse effects of systemic haemostatic medications, such as "unwanted" blood clots. Furthermore, in surgical procedures where the amount of blood loss is unpredictable, topical haemostats can be used sparingly when blood loss is minimal and more liberally during severe bleeding.

A number of topical haemostatic agents are currently available for use in surgery. They can be divided into two categories: those that provide their mechanism of action on the clotting cascade in a biologically active manner and those that act passively through contact activation and promotion of platelet aggregation. Passive topical haemostatic agents include collagens, cellulose, and gelatins, while active agents include thrombin and products in which thrombin is combined with a passive agent to provide an active overall product.

US 2003/0064109 describes a dry haemostatic powder that is prepared by a method comprising: providing an aqueous solution comprising gelatin combined with at least one re-hydration aid; drying the solution to produce solids; grinding the solids to produce a powder; cross-linking the powder; removing at least 50% (w/w) of the re-hydration aid; and drying the cross-linked gelatin to produce a powder. The re-hydration aid may comprise at least one material selected from the group consisting of polyethylene glycol (PEG), polyvinylpyrrolidone (PVP), and dextran.

WO 2010/059280 describes an anhydrous fibrous sheet comprising a first component of fibrous polymer, said polymer containing electrophilic groups or nucleophilic groups, and a second component capable of crosslinking the first component when said sheet is exposed to an aqueous medium in contact with biological tissue to form a crosslinked hydrogel that is adhesive to the biological tissue; wherein:
a) wherein the second component is a fibrous polymer having a backbone structure the same as or different from the fibrous polymer of the first component and containing electrophilic groups if the first component contains nucleophilic groups or containing nucleophilic groups if the first component contains electrophilic groups;
b) the second component is a coating on the fibrous polymer of the first component and wherein said coating contains electrophilic groups if the first component contains nucleophilic groups or nucleophilic groups if the first component contains electrophilic groups; or
c) the second component is a dry powder dispersed and entrapped within interstices of the fibrous polymer of the first component, wherein said powder contains electrophilic groups if the first component contains nucleophilic groups or nucleophilic groups if the first component contains electrophilic groups.

US 2012/0021058 describes a process for making a haemostatic composition, said process comprising: a) providing a dry granular preparation of a biocompatible polymer; and b) coating the granules in said dry granular preparation with a preparation of a coagulation inducing agent, such as a thrombin solution. The biocompatible polymer may be selected from of gelatin, soluble collagen, albumin, hemoglobin, fibrinogen, fibrin, casein, fibronectin, elastin, keratin, laminin, and derivatives or combinations thereof.

US 2013/0316974 describes a haemostatic material comprising a compacted ORC powder comprising particles having average aspect ratio from about 1 to about 18. The haemostatic material may further comprises an additive selected from polysaccharides, calcium salt, antiinfective agent, haemostasis promoting agent, gelatin, collagen,

US 2016/0271228 describes a haemostatic composition comprising:
- a haemostatic biocompatible polymer in particulate form selected from the group consisting of a protein, a polysaccharide, a biologic polymer, a non-biologic polymer, and derivatives and combinations thereof, wherein the haemostatic biocompatible polymer in particulate form is present as granular particles having a median diameter range of 50-700 µm;
- one hydrophilic crosslinker comprising electrophilic reactive groups, wherein the electrophilic reactive groups retain their reactivity until the composition is exposed to blood of the patient, wherein the electrophilic reactive groups are configured to cross-link with blood proteins of the patient to form a gel with sealing and haemostatic properties; and
- a binder that does not react with the electrophilic reactive groups of the one hydrophilic crosslinker;
wherein the haemostatic composition is in paste form.

WO 2012/057628 describes a kit for producing a biocompatible, cross-linked polymer, said kit comprising an electrophilically activated polyoxazoline (EL-POx), said EL-POX comprising m electrophilic groups; and said nucleophilic cross-linking agent comprising n nucleophilic groups, wherein the m electrophilic groups are capable of reaction with the n nucleophilic groups to form covalent bonds; wherein m>2, n>2 and m+n >5; wherein at least one of the m electrophilic groups is a pendant electrophilic group.

WO 2016/056901 describes adhesive haemostatic product selected from a coated mesh, a coated foam or a coated powder, said haemostatic product comprising:
- a porous solid substrate having a porosity of at least 5 vol.% and comprising an outer surface that comprises a nucleophilic polymer containing reactive nucleophilic groups;
- an adhesive coating that covers at least a part of the solid substrate, said coating comprising an electrophilically activated polyoxazoline (EL-POX), said EL-POX containing on average at least 1 reactive electrophilic group.

### SUMMARY OF THE INVENTION

The inventors have developed a haemostatic powder that can conveniently be used to control bleeding during surgery, even for anti-coagulated blood.

The haemostatic powder of the present invention comprises at least 10 wt.% of particle agglomerates, said particle agglomerates having a diameter in the range of 1-500 µm and comprising:
- electrophilic polyoxazoline particles containing electrophilic polyoxazoline carrying at least 3 reactive electrophilic groups that are capable of reacting with amine groups in blood under the formation of a covalent bond; and
- nucleophilic polymer particles containing a water-soluble nucleophilic polymer carrying at least 3 reactive nucleophilic groups that, in the presence of water, are capable of reacting with the reactive electrophilic groups of the electrophilic polyoxazoline under the formation of a covalent bond between the electrophilic polyoxazoline and the nucleophilic polymer.

When applied to a bleeding site, the haemostatic powder of the present invention turns into a gel while at the same time binding to proteins present in the blood and on the surrounding tissue. The haemostatic powder's outstanding ability to stop bleeding is due to highly active induced blood clotting and to the formation of a strong gelled blood clot that adheres to tissue. The haemostatic powder can easily be distributed over a bleeding site. Additional powder can be added if necessary as this will form a new layer of gelled blood clot that will stick to the underlying layer of gelled blood clots.

Although the inventors do not wish to be bound by theory, it is believed that when the haemostatic powder comes into contact with blood, the electrophilic polyoxazoline particles containing electrophilic polyoxazoline rapidly dissolve and simultaneously react with proteins in the blood and reactive nucleophilic groups of the water-soluble nucleophilic polymer in the nucleophilic polymer particles. As a result, a layer of gelled blood clot is formed. The dissolved electrophilic polyoxazoline will also react with proteins in the surrounding tissue at the bleeding site, thereby fixating the layer of gelled blood clot to the tissue and sealing off the bleeding area.

In comparison to particles comprising a molecular mixture of the electrophilic polyoxazoline and the water-soluble nucleophilic polymer, the particle agglomerates of the present invention offer the advantage that they provide better sealing properties. It is believed that this is due to the fact that, when the particle agglomerates come into contact with blood, the electrophilic polyoxazoline reacts with the water-soluble nucleophilic polymer at a relatively slow rate, thereby allowing the electrophilic polyoxazoline to not only react with the water-soluble nucleophilic polymer, but also with proteins in blood and tissue at the bleeding site.

In comparison to a powder mixture consisting of particles of electrophilic polyoxazoline and particles of water-soluble nucleophilic polymer, the particle agglomerates of the present invention offer the advantage that a more homogeneous, strong gel is formed. It is believed that a very homogeneous dispersion of electrophilic polyoxazoline and water-soluble nucleophilic polymer is formed when the particle agglomerates come into contact in blood, and that a homogeneous strong gel is formed when the polymer components dissolve therein and start reacting.

The invention also provides a method of preparing haemostatic particle agglomerates of:
(a) electrophilic polyoxazoline particles containing an electrophilic polyoxazoline carrying at least 3 reactive electrophilic groups that are capable of reacting with amine groups in blood under the formation of a covalent bond; and
(b) nucleophilic polymer particles containing a water-soluble nucleophilic polymer carrying at least 3 reactive nucleophilic groups that are capable of reacting with the reactive electrophilic groups of the electrophilic polyoxazoline under the formation of a covalent bond between the electrophilic polyoxazoline and the nucleophilic polymer;
said method comprising the step of combining 100 parts by weight of the electrophilic polyoxazoline particles with 10 to 1000 parts by weight of the nucleophilic polymer particles in the presence of non-aqueous granulation liquid.

The inventors have unexpectedly discovered that it is possible to combine the electrophilic polyoxazoline and the water-soluble nucleophilic polymer into a single particle with minimum cross-linking reactions between, and minimum degradation of the electrophilic polyoxazoline, by using a non-aqueous granulation liquid in which the electrophilic polyoxazoline is insoluble and in which the nucleophilic polymer is somewhat soluble. Although the inventors do not wish to be bound by theory, it is believed that the use of a non-aqueous granulation liquid in which electrophilic polyoxazoline is insoluble ensures that during granulation no crosslinking reactions will occur between the electrophilic polyoxazoline and the nucleophilic polymer. Also degradation (hydrolysis) of the electrophilic polyoxazoline is minimized in this way. Contrary to the electrophilic polyoxazoline, some of the nucleophilic polymer will dissolve in the non-aqueous granulation liquid, thereby forming a sticky mass that is capable of gluing together the electrophilic polyoxazoline particles and the nucleophilic polymer particles.

Further provided is a biocompatible, flexible, haemostatic sheet comprising:
- a cohesive fibrous carrier structure comprising a three-dimensional interconnected interstitial space; and
- distributed within the interstitial space, the haemostatic powder of the present invention.

### DETAILED DESCRIPTION OF THE INVENTION

Accordingly, a first aspect of the invention relates to a haemostatic powder comprising at least 10 wt.% of particle agglomerates, said particle agglomerates having a diameter in the range of 1-500 µm and comprising:
- electrophilic polyoxazoline particles containing electrophilic polyoxazoline carrying at least 3 reactive electrophilic groups that are capable of reacting with amine groups in blood under the formation of a covalent bond; and
- nucleophilic polymer particles containing a water-soluble nucleophilic polymer carrying at least 3 reactive nucleophilic groups that, in the presence of water, are capable of reacting with the reactive electrophilic groups of the electrophilic polyoxazoline under the formation of a covalent bond between the electrophilic polyoxazoline and the nucleophilic polymer.

The term "particle agglomerate" as used herein refers to a granule that comprises two or more particles that are all bound together.

The term "polyoxazoline" as used herein refers to a poly(N-acylalkylenimine) or a poly(aroylalkylenimine) and is further referred to as POx. An example of POx is poly(2-ethyl-2-oxazoline). The term "polyoxazoline" also encompasses POx copolymers.

The term "water-soluble nucleophilic polymer" as used herein refers to a nucleophilic polymer that has a water-solubility in demineralised water of 20°C, at a pH in the range of 3 to 7, of at least 50 g/L. Chitosan is an example of a water-soluble nucleophilic polymer. Chitosan is water-soluble in water at pH<6. In order to determine water solubility of a nucleophilic polymer at different pH, pH of the demineralised water is adjusted using hydrochloric acid.

The term "collagen" as used herein refers the main structural protein in the extracellular space of various connective tissues in animal bodies. Collagen forms a characteristic triple helix of three polypeptide chains. Depending upon the degree of mineralization, collagen tissues may be either rigid (bone) or compliant (tendon) or have a gradient from rigid to compliant (cartilage). Unless indicated otherwise, the term "collagen" also encompasses modified collagens other than gelatin (e.g. crosslinked collagen).

The term "gelatin" as used herein refers to a mixture of peptides and proteins produced by partial hydrolysis of collagen extracted from the skin, bones, and connective tissues of animals such as domesticated cattle, chicken, pigs, and fish. During hydrolysis, the natural molecular bonds between individual collagen strands are broken down into a form that rearranges more easily. The term "gelatin" as used herein also encompasses modified gelatins, such a crosslinked gelatins and reduced crosslinked gelatins.

The term "reduced crosslinked gelatins" as used herein refers to a crosslinked gelatin that has been partly hydrolysed. Partial hydrolysis of the peptide bonds in the cross-linked gelatin can be achieved by e.g. alkaline treatment. Hydrolysis of the cross-linked gelatin results in an increased density of free carboxyl and free amine groups and in increased water solubility.

The term "haemostatic sheet" as used herein, unless indicated otherwise, refers to a sheet having the ability to stop bleeding from damaged tissue. The haemostatic sheet of the present invention may achieve haemostasis by turning blood into a gel and/or by forming a seal that closes off the wound site.

The term "water-resistant" as used herein in relation to the fibrous carrier structure means that this structure is not water soluble and does not disintegrate in water to form a colloidal dispersion, at neutral pH conditions (pH 7) and a temperature of 37°C.

The term "interstitial space" as used herein refers to the void ("empty") space within the fibrous carrier structure. The interstitial space within the fibrous carrier structure allows the introduction of haemostatic powder into the structure. Also blood and other bodily fluids can enter the interstitial space, thereby allowing the haemostatic powder to exert its haemostatic effect and/or to provide tissue-adhesiveness to the haemostatic sheet.

The term "protein" as used herein, unless indicated otherwise, also encompasses crosslinked and hydrolysed proteins. Likewise, unless indicated otherwise, whenever reference is made to a particular protein species, such as gelatin or collagen, also hydrolysed and crosslinked versions of that protein species are encompassed.

The diameter distribution of the haemostatic powder, of the particle agglomerates, of the electrophilic polyoxazoline particles and of the nucleophilic polymer particles may suitably be determined by means of laser diffraction using a Malvern Mastersizer 2000 in combination with the Stainless Steel Sample Dispersion Unit. The sample dispersion unit is filled with approx. 120 ml of cyclohexane, which is stabilized for 5 to 10 minutes at a stirring speed of 1800 rpm, followed by a background measurement (blanc measurement). The sample tube is shaken and turned horizontally for 20 times. Next, about 50 mg is dispersed in the sample dispersion unit containing the cyclohexane. After the sample is introduced in the dispersion unit, the sample is stirred for one and a half minute at 1800 rpm to ensure that all particles are properly dispersed, before carrying out the measurement. No ultrasonic treatment is performed on the dispersed particles. Mean particle size is expressed as D [4,3], the volume weighted mean diameter (ΣniDi⁴)/(ΣniDi³).

Besides the particle agglomerates comprising the electrophilic polyoxazoline particles and the nucleophilic polymer particles, the haemostatic powder of the present invention may contain other particulate components, e.g. biocompatible (bio)polymers like gelfoam or starch. Preferably, the haemostatic powder contains at least 30 wt.%, more preferably at least 60 wt.% and most preferably at least 80 wt.% of the particle agglomerates.

The particle agglomerates in the haemostatic powder preferably contain at least 10 wt.%, more preferably at least 25 wt.% and most preferably at least 40 wt.% of the electrophilic polyoxazoline.

The nucleophilic polymer is preferably is contained in the particle agglomerates in a concentration of at least 10 wt.%, more preferably of at least 12 wt.% and most preferably of at least 30 wt.%.

The combination of the electrophilic polyoxazoline and the nucleophilic polymer typically constitutes at least 10 wt.% of the particle agglomerates. More preferably, the combination of these two polymers constitutes at least 50 wt.%, most preferably at least 75 wt.% of the particle agglomerates.

Besides the electrophilic polyoxazoline and the nucleophilic polymer the particle agglomerates may contain one or more other components, e.g. polysaccharides. Examples of polysaccharides that may be employed in include amylose, maltodextrin, amylopectin, starch, dextran, hyaluronic acid, heparin, chondroitin sulfate, dermatan sulfate, heparan sulfate, keratan sulfate, dextran sulfate, pentosan polysulfate, alginate and combinations thereof. Typically, the amount of polysaccharide in the particle agglomerates does not exceed 50 wt.%. If polysaccharide is contained in the particle agglomerates, the combination of the electrophilic polyoxazoline, the nucleophilic polymer and the polysaccharide preferably constitutes at least 60 wt.%, more preferably at least 80 wt.% and most preferably at least 90 wt.% of the particle agglomerates.

In accordance with another advantageous embodiment the particle agglomerates contain a dry buffering system. Preferably, the buffering system has a buffering pH in the range of 7 to 11, more preferably in the range of 8 to 10. Preferably, the buffering system has a buffer capacity of at least 10 mmol.l⁻¹.pH⁻¹. More preferably, the buffer capacity is a least 25 mmol.l⁻¹.pH⁻¹, most preferably the buffer capacity is at least 50 mmol.l⁻¹.pH⁻¹. Examples of biocompatible buffering systems that may be employed in the particle agglomerates include sodium phosphate /sodium carbonate buffer; sodium borate decahydrate buffer; tris buffered protein; HEPES buffered saline and sodium bicarbonate/carbonate buffer.

As will be explained below, the particle agglomerates of the present invention can be prepared without the use of a granulation binder, i.e. a component that is employed during granulation to provide adhesion between the electrophilic polyoxazoline particles and the nucleophilic polymer particles. Accordingly, in a preferred embodiment, the particle agglomerates do not contain a granulation binder.

The haemostatic powder of the present invention preferably contains at least 10 wt.% of the particle agglomerates having a diameter in the range of 1-200 µm, more preferably at least 10 wt.% of the particle agglomerates having a diameter in the range of 1-100 µm, and most preferably at least 10 wt.% of the particle agglomerates having a diameter in the range of 1-75 µm

The haemostatic powder preferably has a volume weighted mean diameter (D [4,3], (ΣniD ⁴)/(ΣniD³)) in the range of 10-1000 µm, more preferably in the range of 15-500 µm, most preferably in the range of 30-300 µm.

The particle agglomerates in the haemostatic powder preferably have a D [4,3] in the range of 10-200 µm, more preferably in the range of 15-100 µm, most preferably in the range of 30-60 µm.

The electrophilic polyoxazoline particles in the particle agglomerates preferably have a volume weighted mean diameter (D [4,3]) in the range of 1-100 µm, more preferably in the range of 50-50 µm, most preferably in the range of 10-40 µm.

The nucleophilic polymer particles in the particle agglomerates preferably have a volume weighted mean diameter (D [4,3]) is in the range of 10-300 µm, more preferably in the range of 15-200 µm, most preferably in the range of 20-100 µm.

The electrophilic polyoxazoline particles preferably contain at least 30 wt.%, more preferably at least 50 wt.% and most preferably at least 80 wt.% of the electrophilic polyoxazoline.

The electrophilic polyoxazoline preferably has a solubility in distilled water of 20°C of at least 100 g/L, more preferably of at least 300 g/L.

The electrophilic polyoxazoline preferably has a solubility in isopropyl alcohol at 20°C of less than 10 mg/L, more preferably of less than 5 mg/L and most preferably of less than 1 mg/L.

The electrophilic polyoxazoline preferably has a molecular weight of at least 2 kDa. More preferably, the electrophilic polyoxazoline has a molecular weight of 5 to 200 kDa, most preferably of 10 to 100 kDa.

As explained herein before, the inventors have found a way to combine the electrophilic polyoxazoline and the water-soluble nucleophilic polymer into a single particle with minimum cross-linking reactions between, and minimum degradation of the electrophilic polyoxazoline. Accordingly, in a very preferred embodiment of the invention, the electrophilic polyoxazoline the particle agglomerates has a polydispersity index (PDI) of less than 2.0, more preferably of less than 1.8 and most preferably of less than 1.5.

The electrophilic polyoxazoline preferably contains at least 4 reactive electrophilic groups, more preferably at least 8 reactive electrophilic groups, even more preferably at least 16 reactive electrophilic groups and most preferably at least 32 reactive electrophilic groups.

The electrophilic polyoxazoline typically carries on average at least 10, more preferably at least 20 reactive electrophilic groups.

The electrophilic polyoxazoline is preferably derived from a polyoxazoline whose repeating units are represented by the following formula (I):

(CHR¹)ₘNCOR²

wherein R², and each of R¹ are independently selected from H, optionally substituted C₁₋₂₂ alkyl, optionally substituted cycloalkyl, optionally substituted aralkyl, optionally substituted aryl; and m being 2 or 3.

Preferably, R¹ and R² in formula (I) are selected from H and C₁₋₈ alkyl, even more preferably from H and C₁₋₄ alkyl. R¹ most preferably is H. The integer m in formula (I) is preferably equal to 2.

According to a preferred embodiment, the polyoxazoline is a polymer, even more preferably a homopolymer of 2-alkyl-2-oxazoline, said 2-alkyl-2-oxazoline being selected from 2-methyl-2-oxazoline, 2-ethyl-2-oxazoline, 2-propyl-2-oxazoline, 2-butyl-2-oxazoline and combinations thereof. Preferably, the polyoxazoline is a homopolymer of 2-propyl-2-oxazoline or 2-ethyl-oxazoline. Most preferably, the polyoxazoline is a homopolymer of 2-ethyl-oxazoline.

According to a particularly preferred embodiment, the electrophilic polyoxazoline comprises at least 20 oxazoline units, more preferably at least 30 oxazoline units and most preferably at least 80 oxazoline units. The electrophilic polyoxazoline preferably comprises on average at least 0.05 reactive electrophilic groups per oxazoline residue. Even more preferably, the electrophilic polyoxazoline comprises on average at least 0.1 reactive electrophilic groups per oxazoline residue. Most preferably, the electrophilic polyoxazoline comprises on average 0.12-0.5 reactive electrophilic groups per oxazoline residue.

Polyoxazoline can carry reactive electrophilic groups in its side chains (pendant reactive electrophilic groups), at its termini, or both. The electrophilic polyoxazoline that is employed in accordance with the present invention advantageously contains one or more pendant reactive electrophilic groups. Typically, the electrophilic polyoxazoline contains 0.03-0.5 pendant reactive electrophilic groups per monomer, more preferably 0.04-0.35 pendant reactive electrophilic groups per monomer, even more preferably 0.05-0.25 pendant reactive electrophilic groups per monomer.

In accordance with a preferred embodiment, the reactive electrophilic groups of the electrophilic polyoxazoline are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, olefins, glycidyl ethers, carboxyl, succinimidyl esters, sulfo succinimidyl esters, maleimido (maleimidyl), ethenesulfonyl, imido esters, aceto acetate, halo acetal, orthopyridyl disulfide, dihydroxy-phenyl derivatives, vinyl, acrylate, acrylamide, iodoacetamide and combinations thereof. More preferably, the reactive electrophilic groups are selected from carboxylic acid esters, sulfonate esters, phosphonate esters, pentafluorophenyl esters, p-nitrophenyl esters, p-nitrothiophenyl esters, acid halide groups, anhyinidrides, ketones, aldehydes, isocyanato, thioisocyanato, isocyano, epoxides, activated hydroxyl groups, glycidyl ethers, carboxyl, succinimidyl esters, sulfo succinimidyl esters, imido esters, dihydroxy-phenyl derivatives, and combinations thereof. Even more preferably, the reactive electrophilic groups are selected from halo acetals, orthopyridyl disulfide, maleimides, vinyl sulfone, dihydroxyphenyl derivatives, vinyl, acrylate, acrylamide, iodoacetamide, succinimidyl esters and combinations thereof. Most preferably, the reactive electrophilic groups are selected from maleimides, vinyl, acrylate, acrylamide, succinimidyl esters, sulfo succinimidyl esters and combinations thereof.

Examples of succinimidyl esters that may be employed include succinimidyl glutarate, succinimidyl propionate, succinimidyl succinamide, succinimidyl carbonate, disuccinimidyl suberate, bis(sulfosuccinimidyl) suberate, dithiobis(succinimidylpropionate), bis(2-succinimidooxycarbonyloxy) ethyl sulfone, 3,3'-dithiobis(sulfosuccinimidyl-propionate), succinimidyl carbamate, sulfosuccinimidyl(4-iodoacetyl)aminobenzoate, bis(sulfosuccinimidyl) suberate, sulfosuccinimidyl-4-(N-maleimidomethyl)-cyclohexane-l-carboxylate, dithiobis-sulfosuccinimidyl propionate, disulfo-succinimidyl tartarate; bis[2-(sulfosuccinimidyloxycarbonyloxyethylsulfone)], ethylene glycol bis(sulfosuccinimiclylsuccinate), dithiobis-(succinimidyl propionate).

Examples of dihydroxyphenyl derivatives that may be employed include dihydroxyphenylalanine, 3,4-dihydroxyphenylalanine (DOPA), dopamine, 3,4-dihydroxyhydroccinamic acid (DOHA), norepinephrine, epinephrine and catechol.

The water-soluble nucleophilic polymer that is contained in the nucleophilic polymer particles preferably has a solubility in demineralised water of 20°C, at a pH in the range of 3 to 7, of at least 100 g/L, more preferably of at least 150 g/L and most preferably of at least 200 g/L. The present invention may suitably employ a nucleophilic polymer that is soluble at acidic pH if the electrophilic polyoxazoline releases acidic substances when it reacts with blood components, tissue and/or the nucleophilic polymer. This is the case, for instance, when the electrophilic polyoxazoline contains N-hydroxysuccinimide groups.

The nucleophilic polymer particles preferably contain at least 30 wt.%, more preferably at least 50 wt.% and most preferably at least 80 wt.% of the nucleophilic polymer.

According to a particularly preferred embodiment, the water-soluble nucleophilic polymer that is employed in the agglomerate particles dissolves relatively slowly in water. As explained herein before, it is believed that when the electrophilic polyoxazoline reacts with the water-soluble nucleophilic polymer at a relatively slow rate, the electrophilic polyoxazoline can also react with proteins in blood and tissue at the bleeding site. By employing a water-soluble nucleophilic polymer that dissolves relatively slowly, dissolved electrophilic polyoxazoline has the opportunity to react with proteins in blood and tissue as well as with the (slow) water-soluble nucleophilic polymer, thereby creating a strong homogeneous sealing gel.

Water-soluble nucleophilic polymers having a high molecular weight tend to dissolve relatively slowly in water. Accordingly, in a very preferred embodiment, the water-soluble nucleophilic polymer that is contained in the nucleophilic polymer particles has a molecular weight of at least 3 kDa, more preferably of at least 10 kDa and most preferably of 20 to 3,000 kDa.

The nucleophilic polymer preferably contains at least 4 reactive nucleophilic groups, more preferably at least 8 reactive nucleophilic groups and most preferably at least 10 reactive nucleophilic groups. Most preferably, these reactive nucleophilic groups are amine groups, most preferably primary amine groups.

Examples of water-soluble nucleophilic polymers that can suitably be used in the nucleophilic polymer particles include protein, chitosan, nucleophilic polyoxazoline, nucleophilic polyethylene glycol, polyethyleneimine and combinations thereof. More preferably, the nucleophilic polymer is selected from gelatin, collagen, chitosan, nucleophilic polyoxazoline and combinations thereof.

According to a particularly advantageous embodiment, the nucleophilic polymer particles employed in accordance with the invention provide haemostasis by accelerating the coagulation process. Collagen is capable of activating the intrinsic pathway of the coagulation cascade. Collagen has a large surface area, which acts as a matrix for platelet activation, aggregation, and thrombus formation. Gelatin particles have the ability to restrict blood flow and to provide a matrix for clot formation. Accordingly, in a very preferred embodiment, the nucleophilic polymer is selected from gelatin, collagen and combinations thereof. Most preferably, the nucleophilic polymer is gelatin, even more preferably crosslinked gelatin.

The crosslinked gelatin, preferably has a molecular weight in the range of 30-3,000 kDa, more preferably in the range of 400 to 2,000 kDa, most preferably in the range of 500 to 1,500 kDa.

The average primary amine content of the crosslinked gelatin is in the range of 5 × 10⁻⁴ to 2 × 10⁻² µmol, more preferably 1.0 × 10⁻³ to 1.0 × 10⁻² µmol of primary amine per µg of reduced crosslinked gelatin.

According to another advantageous embodiment, the nucleophilic polymer is nucleophilic polyethylene glycol (PEG). Preferably, the nucleophilic PEG contains at least 3 more preferably at least 5 and most preferably 8 reactive nucleophilic groups

In accordance with a further preferred embodiment, the nucleophilic polymer is chitosan. Chitosan is a biodegradable, nontoxic, complex carbohydrate derivative of chitin (poly-N-acetyl-D-glucosamine), a naturally occurring substance. Chitosan is the deacetylated form of chitin. The chitosan applied in accordance with the present invention preferably has a degree of deacetylation of more than 70%. Chitosan employed in accordance with the present invention preferably has a molecular weight of at least 5 kDa, more preferably of 10-10,000 kDa.

According to another very advantageous embodiment, the nucleophilic polymer is nucleophilic polyoxazoline. Preferably, the nucleophilic polyoxazoline contains at least 3 more preferably at least 5 and most preferably 8 to 20 reactive nucleophilic groups.

In comparison to naturally occurring nucleophilic biopolymers such as gelatin, collagen and chitosan, the use of synthetic nucleophilic polymers, such as nucleophilic polyoxazoline and nucleophilic PEG, offers the advantage that the particle agglomerates containing these synthetic nucleophilic polymers exhibit more predictable (reproducible) haemostatic properties.

The reactive nucleophilic groups of the water-soluble nucleophilic polymer are preferably selected from amine groups, thiol groups and combinations thereof.

According to a preferred embodiment, the water-soluble nucleophilic polymer contains two or more amine groups and the reactive electrophilic groups in the electrophilic polyoxazoline are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, glycidyl ethers, carboxyl, succinimidyl esters, sulfosuccinimidyl esters, imido esters, dihydroxy-phenyl derivatives, and combinations thereof.

According to another preferred embodiment, the water-soluble nucleophilic polymer contains two or more thiol groups and the reactive electrophilic groups of the electrophilic polyoxazoline are selected from halo acetals, orthopyridyl disulfide, maleimides, vinyl sulfone, dihydroxyphenyl derivatives, vinyl, acrylate, acrylamide, iodoacetamide, succinimidyl esters, sulfosuccinmidyl esters and combinations thereof. More preferably, the reactive electrophilic groups are selected from succinimidyl esters, sulfosuccinimidyl esters, halo acetals, maleimides, or dihydroxyphenyl derivatives and combinations thereof. Most preferably, the reactive electrophilic groups are selected from maleimides or dihydroxyphenyl derivatives and combinations thereof.

According to a particularly preferred embodiment, the ratio between the total number of reactive electrophilic groups provided by the electrophilic polyoxazoline and the total number of reactive nucleophilic groups provided by the nucleophilic polymer lies in the range of 1:1.5 to 1.5:1, more preferably in the range of 1:1.2 to 1.2:1 and most preferably in the range of 1:1.1 to 1.1:1.

The inventors have unexpectedly discovered that particle agglomerates showing excellent adhesion to organs such as spleen and kidney can be obtained if the particle agglomerates contain 1-20 wt.%, more preferably 2.5-15 wt.% and most preferably 5-10 wt.% of a non-reactive non-ionic polymer. This non-reactive non-ionic polymer does not contain reactive electrophilic groups or reactive nucleophilic groups.

In a very preferred embodiment, the agglomerated particles are coated with the non-reactive non-ionic polymer.

The non-reactive non-ionic polymer preferably has a melting point in the range of 40-70°C, more preferably in the range of 45-65°C and most preferably in the range of 50-60°C. Here the melting point refers to the temperature at which the polymer is completely melted.

Examples of non-reactive non-ionic polymers that may suitably be applied in the agglomerate particles of the present invention include poloxamers, polyethylene glycols and combinations thereof. Poloxamer is a non-ionic triblock copolymer composed of a central hydrophobic chain of polyoxypropylene (polypropylene oxide)) flanked by two hydrophilic chains of polyoxyethylene (polyethylene oxide)) and is represented by formula (I) 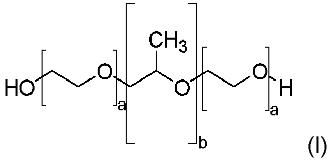 wherein a is an integer of from 10 to 110 and b is an integer of from 20 to 60. When a is 80 and b is 27, this polymer is known as poloxamer 188. Other known poloxamers useful in the present invention are poloxamer 237 (a = 64; and b = 37), poloxamer 338 (a = 141; and b = 44) and poloxamer 407 (a = 101; and b = 56). Further poloxamers that are known and can be useful in the present invention include poloxamer 108, poloxamer 182, poloxamer 183, poloxamer 212, poloxamer 217, poloxamer 238, poloxamer 288, poloxamer 331, poloxamer 338 and poloxamer 335.

According to a particularly preferred embodiment, the non-reactive non-ionic polymer is a poloxamer, even more preferably a poloxamer having an average molecular mass of 2,000-18,000, most preferably a poloxamer having an average molecular mass of 7,000-10,000.

The poloxamer applied in the particle agglomerates preferably is a solid at room temperature.

In another advantageous embodiment, the haemostatic powder of the present invention is bioresorbable, allowing the powder to be used in abdominal surgery.

Another aspect of the invention relates to a method of preparing haemostatic particle agglomerates of:
(a) electrophilic polyoxazoline particles containing an electrophilic polyoxazoline carrying at least 3 reactive electrophilic groups that are capable of reacting with amine groups in blood under the formation of a covalent bond; and
(b) nucleophilic polymer particles containing a water-soluble nucleophilic polymer carrying at least 3 reactive nucleophilic groups that are capable of reacting with the reactive electrophilic groups of the electrophilic polyoxazoline under the formation of a covalent bond between the electrophilic polyoxazoline and the nucleophilic polymer;
said method comprising combining 100 parts by weight of the electrophilic polyoxazoline particles with 10 to 1000 parts by weight of the nucleophilic polymer particles in the presence of non-aqueous granulation liquid.

The electrophilic polyoxazoline particles that are employed in the present method are preferably identical to the electrophilic polymer particles described herein before. Likewise, the nucleophilic polymer particles that are employed are preferably identical to the nucleophilic polymer particles described herein before.

According to a preferred embodiment, both the electrophilic polyoxazoline particles and the nucleophilic polymer particles that are employed in the present method have a water content of less than 2 wt.%, more preferably of less than 1 wt.%. To achieve such a low water content, it may be necessary to dry the polymer particles before the granulation.

The electrophilic polyoxazoline preferably has a solubility in the non-aqueous granulation liquid at 20°C of less than 1 mg/L, more preferably of less than 0.5 mg/L, even more preferably of less than 0.2 mg/L and most preferably of less than 0.1 mg/L.

The nucleophilic polymer preferably has a solubility in the non-aqueous granulation liquid at 20°C of at least 5 mg/L, more preferably of at least 10 mg/L, even more preferably of 20-200 mg/L and most preferably of 40-150 mg/L.

In a preferred embodiment, the method comprises combining 100 parts by weight of the electrophilic polyoxazoline particles with 20 to 400 parts by weight, more preferably 50 to 200 parts by weight of the nucleophilic polymer particles, in the presence of the non-aqueous granulation liquid.

The amount of non-aqueous granulation liquid employed in the present method preferably is in the range of 0.5-5% by weight of the combined amount of electrophilic polyoxazoline particles and nucleophilic polymer particles that is used in the method. More preferably, the amount of non-aqueous granulation liquid employed is in the range of 1-4 %, most preferably 1.5-3 % by weight of the combined amount of electrophilic polyoxazoline particles and nucleophilic polymer particles.

According to a particularly preferred embodiment, the method comprises the step of wetting the electrophilic polyoxazoline particles with the non-aqueous granulation liquid, followed by the step of combining the wetted polyoxazoline particles with the nucleophilic polymer particles. This particular embodiment offers the advantage that it yields a granulate that is very homogeneous in terms of particle size and composition.

The electrophilic polyoxazoline particles used in the preparation of the powder blend preferably have an volume weighted mean diameter (D [4,3]) in the range of 1-100 µm, more preferably in the range of 50-50 µm, most preferably in the range of 10-40 µm.

The volume weighted mean diameter (D [4,3]) of the nucleophilic polymer particles used in the preparation of the powder blend preferably is in the range of 10-300 µm, more preferably in the range of 15-200 µm, most preferably in the range of 20-100 µm.

The agglomerated powder that is obtained by the present method preferably has a volume weighted mean diameter (D [4,3]) in the range of 12-1000 µm, more preferably in the range of 20-500 µm, most preferably in the range of 30-300 µm.

The non-aqueous granulation liquid employed in the wet granulation preferably contains at least 60 wt.% of an organic solvent selected from isopropyl alcohol, ethanol, methanol, diethyl ether, heptane, hexane, pentane, cyclohexane, dichloromethane, acetone and mixtures thereof. More preferably, the non-aqueous granulation liquid contains at least 60 wt.%, most preferably at least 85 wt.% of an organic solvent selected from isopropyl alcohol, ethanol and mixtures thereof. Even more preferably, the non-aqueous granulation liquid contains at least 60 wt.%, most preferably at least 85 wt.% of isopropyl alcohol.

The non-aqueous granulation liquid preferably contains not more than 1 wt.% water, more preferably not more than 0.1 wt.% water.

The present method offers the advantage that it does not require the use of granulation binder. Accordingly, in a preferred embodiment of the method, no granulation binder is used. According to a particularly preferred embodiment, the only materials used in the preparation method are the electrophilic polyoxazoline particles, the nucleophilic polymer particles and the non-aqueous granulation liquid.

The particle agglomerates of the present invention may advantageously be applied in haemostatic sheets to improve the haemostatic properties thereof. Accordingly, yet another aspect (not part of the claimed invention) relates to a biocompatible, flexible, haemostatic sheet comprising:
- a cohesive fibrous carrier structure comprising a three-dimensional interconnected interstitial space; and
- distributed within the interstitial space, a haemostatic powder as described herein before.

In accordance with a particularly preferred embodiment, the cohesive fibrous carrier structure is water resistant.

The electrophilic polyoxazoline in the haemostatic powder that is distributed throughout the fibrous carrier structure dissolve rapidly when the sheet comes into contact with blood or other aqueous bodily fluids that can penetrate the interstitial space. Thus, upon application of the sheet onto a wound site, rapid covalent cross-linking occurs between on the one hand the electrophilic polyoxazoline, and on the other hand the nucleophilic polymer in the nucleophilic polymer particles, blood proteins and tissue, leading to the formation of a gel which seals off the wound surface and stops the bleeding and further leading to strong adhesion of the haemostatic sheet to the tissue The chitosan applied in accordance with the present invention preferably has a degree of deacetylation of more than 70%. The water-resistant fibrous carrier structure provides mechanical strength during and after application, and prevents excessive swelling.

Since the electrophilic polyoxazoline and the nucleophilic polymer are contained in a single particle, it is ensured that these two reactive components can be homogeneously distributed throughout the haemostatic sheet, that no segregation occurs during transport and handling, and that these components can react immediately with each other when the particle agglomerates come into contact with blood.

According to a particularly preferred embodiment, the haemostatic sheet of the present invention is bioabsorbable, meaning that the carrier structure, the particle agglomerates and any other components of the haemostatic sheet are eventually absorbed in the body. Absorption of the carrier structure and the particle agglomerates typically requires chemical decomposition (e.g. hydrolysis) of the polymers contained therein. Complete bioabsorption of the haemostatic sheet by the human body is typically achieved in 1 to 10 weeks, preferably in 2 to 8 weeks.

The haemostatic sheet of the present invention typically has a non-compressed mean thickness of 0.5-25 mm. More preferably, the non-compressed mean thickness is in the range of 1-10 mm, most preferably in the range of 1.5-5 mm.

The dimensions of the haemostatic sheet preferably are such that the top and bottom of the sheet each have a surface area of at least 2 cm², more preferably of at least 10 cm² and most preferably of 25-50 cm². Typically, the sheet is rectangular in shape and has a length of 25-200 mm, an a width of 25-200 mm.

The haemostatic sheet preferably has a non-compressed density of less than 200 mg/cm³, more preferably of less than 150 mg/cm³ and most preferably of 10-100 mg/cm³.

The haemostatic sheet preferably is essentially anhydrous. Typically the haemostatic sheet has a water content of not more than 5 wt.%, more preferably of not more than 2 wt.% and most preferably of not more than 1 wt.%.

The water absorption capacity of the haemostatic sheet preferably is at least 50%, more preferably lies in the range of 100% to 800%, most preferably in the range of 200% to 500%.

The haemostatic sheet is preferably sterile.

The use of a fibrous carrier structure in the haemostatic sheet offers the advantage that the haemostatic powder can be homogeneously distributed throughout this carrier structure without difficulty. Such a homogeneous distribution is much more difficult to achieve in, for instance, foamed carrier structures.

The fibres in the fibrous carrier structure preferably have a mean diameter of 1-500 µm, more preferably of 2-300 µm and most preferably of 5-200 µm. The mean diameter of the fibres can suitably be determined using a microscope.

Typically, at least 50 wt.%, more preferably at least 80 wt.% of the fibres in the fibrous carrier structure have a diameter of 1-300 µm and a length of at least 1 mm.

Preferably, at least 50 wt.%, more preferably at least 80 wt.% of the fibres in the fibrous carrier structure have an aspect ratio (ratio of length to diameter) of at least 1000.

The fibrous carrier structure that is employed preferably is a felt structure, a woven structure or a knitted structure. Most preferably, the fibrous carrier structure is a felt structure. Here the term "felt structure" refers to a structure that is produced by matting and pressing fibres together to form a cohesive material.

The fibrous carrier structure preferably comprises at least 50 wt.%, more preferably at least 80 wt.% and most preferably at least 90 wt.% fibres containing gelatin, collagen, cellulose, modified cellulose, carboxymethyldextran, PLGA, sodium hyaluronate/carboxy methylcellulose, polyvinyl alcohol, chitosan or a combination thereof.

In an embodiment of the invention, the fibrous carrier structure does not comprise oxidised regenerated cellulose.

Preferred fibrous carrier structures have an open pore structure with a permeability to air of at least 0.1 L/min x cm², more preferably of at least 0.5 L/min x cm². The air permeability is determined in accordance with EN ISO 9237:1995 (Textiles - Determination of the permeability of fabrics to air).

The fibres in the fibrous carrier structure can be produced by means of methods known in the art, such as electrospinning, electro-blown spinning and high speed rotary sprayer spinning. Production of fibrous carrier structure by means of high speed rotary sprayer spinning is described in US 2015/0010612. It is also possible to use commercially available haemostatic fibrous sheets as the fibrous carrier structure.

The haemostatic powder is preferably present in the haemostatic sheet of the present invention in an amount of 5-90%, more preferably 10-80%, even more preferably 20-75% and most preferably 50-70%, by weight of the fibrous carrier structure.

The invention is further illustrated by the following non-limiting examples.

### EXAMPLES (examples not comprising a powder according to claim 1 are not part of the claimed invention)

In general: wherever residual moisture (i.e., residual water in dried powder, granulate and/or cohesive fibrous carrier structures) after drying is not explicitly mentioned, levels are below 2.0% w/w.

### Preparation of NHS-POx

NHS-side chain activated poly[2-(ethyl/hydroxy-ethyl-amide-ethyl/NHS-ester-ethyl-ester-ethyl-amide-ethyl)-2-oxazoline] terpolymer containing 20% NHS-ester groups (= EL-POx, 20% NHS) was synthesized as follows: Poly[2-(ethyl/methoxy-carbonyl-ethyl)-2-oxazoline] copolymer (DP = +/-100) was synthesized by means of CROP using 60% 2-ethyl-2-oxazoline (EtOx) and 40% 2-methoxycarbonyl-ethyl-2-oxazoline (MestOx). A statistical copolymer containing 40% 2-methoxycarbonyl-ethyl groups (¹H-NMR) was obtained. Secondly, the polymer containing 40% 2-methoxycarbonyl-ethyl groups, was reacted with ethanolamine yielding a copolymer with 40% 2-hydroxy-ethyl-amide-ethyl-groups (¹H-NMR). After that, half of the 2-hydroxy-ethyl-amide-ethyl-groups was reacted with succinic anhydride yielding a terpolymer with 60% 2-ethyl groups, 20% 2-hydroxy-ethyl-amide-ethyl-groups and 20% 2-carboxy-ethyl-ester-ethyl-amide-ethyl-groups according to ¹H-NMR. Lastly, the 2-carboxy-ethyl-ester-ethyl-amide-ethyl-groups were activated by N-hydroxysuccinimide (NHS) and diisopropylcarbodiimide (DIC), yielding EL-POx, 20% NHS. The NHS-POx contained 20% NHS-ester groups according to ¹H-NMR. NHS-POx was dissolved between 2-8 °C in water (60 g in 300 mL), cooled at minus 80 °C for half an hour and freeze dried. The freeze dried powder so obtained was dried in a Rotavap at 40°C until the water content was below 0.8% w/w as determined via Karl Fischer titration. This dry (white) powder was grinded using a ball mill (Retch MM400) until the average particle size was not more than 40 µm (D [4,3]) and vacuum sealed in alu-alu bags.

### Dying of NHS-POx powder

20 g of NHS-POx powder were dissolved in water and mixed with 50 mg Brilliant Blue FCF (Sigma Aldrich) using a high-performance dispersing instrument (Ultra-Turrax, IKA). Directly after mixing (2 minutes) the solution was frozen at -78 °C and subsequently freeze dried overnight. The freeze dried powder so obtained was dried in a Rotavap at 40 °C until the residual water content was below 0.8% w/w as determined via Karl Fischer titration. Next, the dried (blue) powder was grinded using a ball mill (Retch MM400) until a blue dyed NHS-POx powder having an average particle size of not more than 40 µm (D [4,3]) and vacuum sealed in alu-alu bags.

### Effect of crosslinking of NHS-POx on PDI (polydispersity index)

A 80 µg/mL solution of 2,2'-(ethylenedioxy)-bis-(ethylamine) (EDEA, Aldrich, Mw 148.2) was prepared in a mixture of dichloromethane (DCM) and isopropanol (IPA) by dissolving 80 mg of EDEA in 10 mL of DCM/IPA 95:5 (v/v). This solution was diluted 10 times with DCM/IPA 95:5 (v/v) and 26.8 µL of the EDEA solution were added to 100 µL of a 0.5 g/mL solution of NHS-POx in DCM/IPA 95:5 (v/v) corresponding with 0.05 mole% amine groups with respect to NHS groups. Directly after addition of the EDEA solution, the reaction mixture was thoroughly mixed using a vortex mixer. The reaction mixture was agitated at 40°C for 3 hours and all volatiles were removed by rotary evaporation under reduced pressure.

The dried sample was reconstituted in the size exclusion chromatographic (SEC) mobile phase *N,N*-dimethylacetamide containing 50 mM lithium chloride. SEC was measured against poly(methyl methacrylate) standards. From the obtained size exclusion chromatogram, the Mₙ, M_{w} and PDI were determined. The PDI was more than 2.5 A 0.5 g/mL solution of NHS-POx in DCM/IPA 95:5 (v/v), without the addition of EDEA, following the same SEC procedure resulted in a PDI of 1.5, indicating that only 0.05 mole% crosslinking of NHS groups already results in a significant increase in PDI of the polymer.

### Preparation of NU-POx

Polyoxazoline containing ethyl and amine groups in the alkyl side chain was synthesized by CROP of EtOx and MestOx and subsequent amidation of the methyl ester side chains with ethylene diamine to yield a poly(2-ethyl/aminoethylamidoethyl-2-oxazoline) copolymer (NU-POx). The NU-POx contained 10% NH₂ according to ¹H-NMR. NU-POx was dissolved between 2-8 °C in water (60 g in 300 mL), cooled at minus 80 °C for half an hour an freeze dried. The freeze dried powder so obtained was dried in a Rotavap at 40 °C until the water content was below 0.8% w/w as determined via Karl Fischer titration. This dry powder was grinded in a table top grinding machine until the average particle size was not more than 100 µm (D [4,3]) and vacuum sealed in alu-alu bags.

### Preparation of reactive NHS-POx / NU-POx granules

Blue or white (non-dyed) NHS-POx powder was wetted with isopropyl alcohol (IPA) in a high shear mixer until a homogeneous snow-like powder was obtained containing about 1-2% w/w IPA. After this, NU-POx powder was added and mixed. The wetted blue NHS-POx powder was mixed with NU-POx powder in a molar ratio of 1:0.6, said molar ratio referring to the ratio of the number of NHS groups provided by NHS-POx to the number of amine groups provided by the NU-POx. The wetted non-dyed NHS-POx powder was also mixed with NU-POx powder in other molar ratios (1:0.8; 1:1 and 1:1.2).

After mixing, the wet granulates were dried under reduced pressure until the IPA content was less than 0.1% w/w as determined via ¹H-NMR. The dried granulates were grinded using a ball mill (Retch MM400) until the average particle size was not more than 50 µm (D [4,3]) and vacuum sealed in alu-alu bags.

The particle size distribution of the granulates so obtained was approximately: 90 vol.% < 90 µm, 50 vol.% < 45 µm and 10 vol.% < 15 µm.

The NHS-POX/NU-POx granulate (1:1) was analysed using ¹H-NMR spectroscopy. 25 mg of granulate were dissolved in trifluoroacetic acid (0.20 mL) by sonicating for 20 minutes. After complete dissolution of the granulate, the sample was diluted with deuterated dimethylsulfoxide (DMSO-*d₆*) containing maleic acid (2.5 mg/mL) as an internal standard (0.80 mL), transferred to an NMR tube and a ¹H-NMR spectrum was recorded. From the acquired spectrum, the amount of NHS bound to NHS-POx can be calculated, along with the molar ratio of NHS and amine groups present in the granulate. The amount of NHS bound to NHS-POx in the granulate was equal to the amount of NHS bound to NHS-POx starting material indicating no decay or cross linking during granulation.

The total polymer recovery, *i.e.* the combination of NHS-POx and NU-POx, in the NMR sample was determined using a known amount of internal standard (maleic acid) and a calibration curve constructed from ¹H-NMR spectra recorded of NHS-POx and NU-POx in different concentrations. The total polymer recovery was measured to be 99 percent, indicating that no insoluble crosslinked material was formed.

The NHS-POX/NU-POx granulate (1:1) was further analysed by means of size exclusion chromatography. 20 mg of the granulate was treated with acetic anhydride (1.00 mL) for 1 hour at 50°C. Subsequently, methanol (2.00 mL) was added and the mixture was stirred for an additional hour at 50°C. An aliquot (0.75 mL) was taken and all volatiles were removed under reduced pressure. The sample was taken up in *N,N*-dimethylacetamide containing 50 mM lithium chloride (2.50 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating no cross linking had occurred during granulation. Analytical validation of this size exclusion chromatographic method indicated that intentional cross linking of NHS-POx with NU-POx at a level of 0.05 mol% increased the PDI to more than 2.5.

### Preparation of NHS-POx / NU-POx mixtures by co-freeze drying

Co-freeze dried NHS-POx/NU-POx powders were prepared as follows: 2.4 g of NU-POx were dissolved in 40 mL of glacial acetic acid. After complete dissolution of the polymer, 2.0 g of NHS-POx were added and the sample was sonicated for 30 minutes. The solution was flash frozen in liquid nitrogen and freeze dried. The resulting sticky solid was further dried under reduced pressure (< 1 kPa) and vacuum sealed in an alu-alu bag.

Co-freeze dried NHS-POx / NU-POx samples could not be analyzed by means of ¹H-NMR spectroscopy and size exclusion chromatography because the samples were neither soluble in trifluoroacetic acid nor acetic anhydride due to a high degree of crosslinking between NHS-POx and NU-POx.

### Preparation of NHS-POx / NU-POx mixtures by dry mixing

NHS-POx/NU-POx mixtures were prepared as follows: 2 g of NHS-POx and 2 g of NU-POx were dry mixed by means of tumble mixing for 30 minutes. The resulting powder was dried under reduced pressure (< 1kPa) and vacuum sealed in an alu-alu bag.

The powder was analysed using ¹H-NMR spectroscopy. 25 mg of powder were dissolved in trifluoroacetic acid (0.20 mL) by sonicating for 20 minutes. After complete dissolution of the powder, the sample was diluted with deuterated dimethylsulfoxide (DMSO-*d₆*) containing maleic acid (2.5 mg/mL) as an internal standard (0.80 mL), transferred to an NMR tube and a ¹H-NMR spectrum was recorded. From the obtained spectrum, the amount of non-reacted NHS was calculated to be 99 percent compared to NHS-POx.

The total polymer recovery, *i.e.* the combination of NHS-POx and NU-POx, in the NMR sample was determined using a known amount of internal standard (maleic acid) and a calibration curve constructed from ¹H-NMR spectra recorded of NHS-POx and NU-POx in different concentrations. The total polymer recovery was measured to be 101 percent, indicating that no insoluble crosslinked material was formed.

The powder was further analysed by means of size exclusion chromatography. 20 mg of the powder was treated with acetic anhydride (1.00 mL) for 1 hour at 50°C. Subsequently, methanol (2.00 mL) was added and the mixture was stirred for an additional hour at 50°C. An aliquot (0.75 mL) was taken and all volatiles were removed under reduced pressure. The sample was taken up in *N,N*-dimethylacetamide containing 50 mM lithium chloride (2.50 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking had occurred during dry mixing.

### Preparation of NHS-POx / NU-POx (sequestered) granules

The following powders were produced by freeze drying:

### Phosphate powder

42.66 g of sodium phosphate dibasic dihydrate and 26.56 g of sodium phosphate monobasic monohydrate were dissolved in 170 mL ultrapure water. After complete dissolution, the pH was adjusted to 7 by addition of 62 mL of a 0.1 molar aqueous sodium hydroxide solution. The solution was frozen in liquid nitrogen and freeze dried . The resulting powder was dried under reduced pressure and vacuum sealed in an alu-alu bag.

### Carbonate powder

A 1:1 mole/mole mixture of sodium carbonate and sodium hydrogen carbonate was prepared by dissolving 25.31 g of sodium carbonate and 20.06 g of sodium hydrogen carbonate in 350 mL ultrapure water. The solution was flash frozen in liquid nitrogen and freeze dried. The resulting powder was dried under reduced pressure and vacuum sealed in an alu-alu bag.

### NHS-POx/ Citric acid powder

3.08 g of citric acid were dissolved in 10 mL ultrapure water. The solution was cooled between 2-8°C. Subsequently, 7.00 g of NHS-POx were added and dissolved with the aid of a high-performance dispersing instrument (Ultra-Turrax, IKA). Directly after mixing (2 minutes), the solution was flash frozen using liquid nitrogen and freeze dried overnight. The resulting powder was dried under reduced pressure and vacuum sealed in an alu-alu bag.

### NU-POx/ Carbonate Powder

Next, a carbonate-containing NU-POx powder was produced as follows:9.60 g of NU-POx and 0.80 g of the carbonate powder were dissolved in 40 mL ultrapure water. The solution was flash frozen using liquid nitrogen and freeze dried. The resulting powder was dried under reduced pressure and vacuum sealed in an alu-alu bag.

NHS-POx / NU-POx (sequestered) granulate was prepared as follows: 6.50 g of NHS-POx/Citric acid powder and 5.50 g of the phosphate powder were mixed in a high shear mixer. After obtaining a homogeneous mixture, 0.40 mL IPA were added slowly, while the mixing was continued, until a homogeneous snow-like powder was formed. Next, 5.41 g of the NU-POx/Carbonate powder were added and the mixing was stopped once a homogeneous granulate was formed. The granulate was dried under reduced pressure pressure until the IPA content was less than 0.1 % w/w. The dried granulate was milled in a coffee grinder until the average particle size was not more than 100 µm (D [4,3]) and vacuum sealed in an alu-alu bag.

### Preparation of reactive NHS-POx / NU-POx / P188 granules

Reactive NHS-POx / NU-POx granules as described previously were coated with Pluronic P188. 2.5% w/w P188 coated reactive NHS-POx / NU-POx granulate was prepared by heating the NHS-POx / NU-POx granulate together with P188 powder in a high shear mixer at 65 °C for 10 minutes followed by cooling down to ambient conditions. The coated granulate was grinded using a ball mill (Retch MM400) until the average particle size was not more than 40 µm (D [4,3]) and vacuum sealed in alu-alu bags.

The particle size distribution of the granulates so obtained was approximately: 90 vol.% < 80 µm, 50 vol.% < 40 µm and 10 vol.% < 10 µm.

The NHS-POx/NU-POx/P188 granulate was analysed using ¹H-NMR spectroscopy. 25 mg of powder were dissolved in trifluoroacetic acid (0.20 mL) by sonicating for 20 minutes. After complete dissolution of the granulate, the sample was diluted with deuterated dimethylsulfoxide (DMSO-*d₆*) (0.80 mL), transferred to an NMR tube and a ¹H-NMR spectrum was recorded. From the obtained spectrum, the amount of non-reacted NHS was calculated to be 98 percent compared to NHS-POx.

The NHS-POx/NU-POx/P188 granulate was further analysed by means of size exclusion chromatography. 20 mg of the granulate was treated with acetic anhydride (1.00 mL) for 1 hour at 50°C. Subsequently, methanol (2.00 mL) was added and the mixture was stirred for an additional hour at 50°C. An aliquot (0.75 mL) was taken and all volatiles were removed under reduced pressure. The sample was taken up in *N,N*-dimethylacetamide containing 50 mM lithium chloride (2.50 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking had occurred during granulation.

### Preparation of reduced crosslinked gelatin (RXL)

Reduced crosslinked gelatin (RXL) was prepared according to three procedures:
- 12 g of gelatin powder (Gelita-SPONO, Gelita Medical GmbH) were dissolved in 350 mL of a 0.1 molar aqueous sodium hydroxide solution by stirring for 2 hours at 40°C. After a clear solution was obtained, the mixture was allowed to cool down to ambient temperature and the pH was adjusted to 7 by addition of 32.5 mL of a 1.0 molar aqueous hydrochloric acid solution. The solution was flash frozen using liquid nitrogen and freeze dried. Subsequently, the powder was milled in a coffee grinder, dried under reduced pressure and vacuum sealed in an alu-alu bag. Hereafter, this reduced crosslinked gelatin will be referred to as RXL-LS (low salt).
- 12 g of gelatin powder (Gelita-SPONO, Gelita Medical GmbH) were dissolved in 360 mL of a 1.0 molar aqueous sodium hydroxide solution by stirring for 10 minutes at 40°C. The obtained clear solution was cooled to ambient temperature and the pH was decreased to 7 by addition of 30 mL of a concentrated hydrochloric acid solution (37 % w/w). The solution was flash frozen using liquid nitrogen and freeze dried. Subsequently, the powder was milled in a coffee grinder, dried under reduced pressure and vacuum sealed in an alu-alu bag. Hereafter, this reduced crosslinked gelatin will be referred to as RXL-HS (high salt).
- 12 g of gelatin powder (Gelita-SPONO, Gelita Medical GmbH) were dissolved in 350 mL of a 0.1 molar aqueous sodium hydroxide solution by stirring for 2 hours at 40°C. After a clear solution was obtained, the mixture was allowed to cool down to ambient temperature and the pH was adjusted to 7 by addition of 32.5 mL of a 1.0 molar aqueous hydrochloric acid solution. Subsequently, 400 mL of methanol was added to the solution which was placed in a freezer at -20°C for 16 hours resulting in precipitation of RXL, which was isolated by decanting the liquid phase. The RXL was washed three times with 100 mL portions of methanol and dried under vacuum afterwards. The crude product was dissolved in 200 mL ultrapure water, flash frozen in liquid nitrogen and freeze dried. The powder was milled in a coffee grinder, dried under reduced pressure and vacuum sealed in an alu-alu bag. Hereafter, this reduced crosslinked gelatin will be referred to as RXL (no salt).

### Preparation of reactive NHS-POx / RXL granules (no salt, low salt and high salt)

NHS-POx/RXL reactive granules were prepared as follows: 5 g of blue NHS-POx powder were wetted with IPA in a high shear mixer until a homogeneous snow like powder was obtained containing about 1-2% w/w IPA. After this, 5 g of RXL, RXL-LS or RXL-HS powder were added and mixed. After mixing, the wet granulates were dried under reduced pressure until the IPA content was less than 0.1% w/w as determined via ¹H-NMR. The dried granulates were milled in a coffee grinder until the average particle size was not more than 90 µm (D [4,3]) and vacuum sealed in alu-alu bags.

The particle size distribution of the granulates so obtained was approximately: 90 vol.% < 190 µm, 50 vol.% < 60 µm and 10 vol.% < 15 µm.

The granulate containing RXL was analysed by means of ¹H-NMR spectroscopy analysis. To this end deuterated chloroform (CDCl3) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 98 percent compared to NHS-POx.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 100 percent, indicating that no insoluble crosslinked material was formed.

The NHS-POx/RXL granulate was further analysed by means of size exclusion chromatography. Therefore, an aliquot (0.15 mL) was taken from the solution used for ¹H-NMR spectroscopy analysis. This solution was diluted with *N,N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was not more than 1.5, again indicating that no cross linking had occurred during granulation.

### Preparation of NHS-POx / RXL mixtures by co-freeze drying

Co-freeze dried NHS-POx/RXL powders were prepared as follows: 2.5 g of RXL powder were dissolved in 200 mL of ultrapure water. The pH was adjusted to 4.5 by the addition of acetic acid and the solution was cooled to 4°C. Subsequently, 2.5 g of NHS-POx was added and dissolved with the aid of high shear stirring. Directly after complete dissolution of the NHS-POx, the solution was flash frozen in liquid nitrogen and freeze dried. The resulting powder was dried under reduced pressure and vacuum sealed in an alu-alu bag.

The co-freeze dried NHS-POx/RXL powder was analysed by means of ¹H-NMR spectroscopy analysis. To this end deuterated chloroform (CDCl₃) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 93 percent compared to NHS-POx.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 81 percent, indicating that to some extent insoluble crosslinked material was formed.

The NHS-POx/RXL powder was further analysed by means of size exclusion chromatography. An aliquot (0.15 mL) was taken from the solution used for ¹H-NMR spectroscopy analysis. This solution was diluted with *N,N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was 3.3 indicating that cross linking had occurred during co-freeze drying of both components.

### Preparation of NHS-POx / RXL mixtures by dry mixing

NHS-POx/RXL mixtures were prepared as follows: 2 g of RXL powder and 2 g of NHS-POx were dry mixed by means of tumble mixing for 30 minutes. The resulting powder was dried under reduced pressure and vacuum sealed in an alu-alu bag.

The powder was analysed by means of ¹H-NMR spectroscopy analysis. To this end deuterated chloroform (CDCl₃) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 100 percent compared to NHS-POx.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 104 percent, indicating that no insoluble crosslinked material was formed.

The NHS-POX/RXL powder was further analysed by means of size exclusion chromatography. Therefore, an aliquot (0.15 mL) was taken from the solution used for ¹H-NMR spectroscopy analysis. This solution was diluted with *N,N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking had occurred during dry mixing.

### Preparation of reactive NHS-POx / RXL granules containing carbonate

First, a 1:1 mole/mole mixture of sodium carbonate and sodium hydrogen carbonate was prepared by dissolving 25.31 g of sodium carbonate and 20.06 g of sodium hydrogen carbonate in 350 mL ultrapure water. The solution was flash frozen in liquid nitrogen and freeze dried. The resulting powder was dried under reduced pressure and vacuum sealed in an alu-alu bag.

The NHS-POx/RXL/carbonate granulates were prepared as follows: 5 g of RXL-LS or RXL-HS and 0.178 g of the sodium carbonate / sodium hydrogen carbonate were mixed using a high shear mixer. Next, 5 g of blue NHS-POx were added containing about 1-2% w/w IPA and mixed until a homogeneous powder was obtained. After mixing, the wet granulates were dried under reduced pressure until the IPA content was less than 0.1% w/w as determined via ¹H-NMR. The dried granulates were milled in a coffee grinder until the average particle size was not more than 100 µm (D [4,3]) and vacuum sealed in alu-alu bags.

### Preparation of reactive NHS-POx / NH2-PEG granules

NHS-POx (6,9 g) was wetted with IPA in a high shear mixer until a homogeneous snow-like powder was obtained containing about 1-2% w/w IPA. Subsequently, 8.1 g of amine-PEG-amine, 2-arm, MW 2k (ex Creative PEGWorks) were added (molar ratio of 1:1.16, said molar ratio referring to the ratio of the number of NHS groups provided by NHS-POx to the number of amine groups provided by the PEG-amine). The formed granulate was dried under reduced pressure until the IPA content was less than 0.1 % w/w as determined via ¹H-NMR. The dried granulate was milled in a coffee grinder until the average particle size was not more than 100 µm (D [4,3]) and vacuum sealed in alu-alu bags.

The NHS-POX/NH2-PEG granulate was analysed using ¹H-NMR spectroscopy. 25 mg of granulate were dissolved in trifluoroacetic acid (0.20 mL) by sonicating for 20 minutes. After complete dissolution of the granulate, the sample was diluted with deuterated dimethylsulfoxide (DMSO-*d₆*) (0.80 mL), transferred to an NMR tube and a ¹H-NMR spectrum was recorded. From the obtained spectrum, the amount of non-reacted NHS was calculated to be 97 percent compared to NHS-POx.

The NHS-POX/NH2-PEG granulate was further analysed by means of size exclusion chromatography. 20 mg of the granulate was treated with acetic anhydride (1.00 mL) for 1 hour at 50°C. Subsequently, methanol (2.00 mL) was added and the mixture was stirred for an additional hour at 50°C. An aliquot (0.75 mL) was taken and all volatiles were removed under reduced pressure. The sample was taken up in *N,N*-dimethylacetamide containing 50 mM lithium chloride (2.50 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking occurred during granulation.

### Preparation of starch / NHS-POx / NU-POx granules

The following starch powders were produced by freeze drying:

### NHS-POx / Starch powder

2.79 g of starch (Arista^{™} AH, BARD) was dispersed in 50 mL of ultrapure water using a high shear mixer. The dispersion was cooled between 2-8°C and 0.71 g of NHS-POx were added and dissolved with the aid of high shear mixing.

Directly after dissolution of the NHS-POx, the solution was flash frozen using liquid nitrogen and freeze dried. The resulting powder was dried under reduced pressure and vacuum sealed in an alu-alu bag.

### NU-POx / Starch powder

1.45 g of starch (Arista^{™} AH, BARD) were dispersed in 30 mL of ultrapure water using a high shear mixer. Subsequently, 0.36 g of NU-POx were added and allowed to dissolve. After complete dissolution of the NU-POx, the dispersion was flash frozen using liquid nitrogen and freeze dried. The resulting powder was dried under reduced pressure and vacuum sealed in an alu-alu bag.

### NU-POx / Starch / Sodium Carbonate powder

2.25 g of starch (Arista^{™} AH, BARD) were dispersed in 50 mL of ultrapure water using a high shear mixer. Subsequently, 0.58 g of NU-POx and 0.25 g of sodium carbonate were added and allowed to dissolve. After complete dissolution of the NU-POx, the dispersion was flash frozen using liquid nitrogen and freeze dried. The resulting powder was dried under reduced pressure and vacuum sealed in an alu-alu bag.

Two granulates were prepared from these three powders. Starch / NHS-POx / NU-POx granulates were prepared as follows:

### Starch Granulate 1:

1.41 g of the NHS-POx / Starch powder, 1.68 g of the NU-POx / Starch powder and 0.20 mL of IPA were mixed using a pestle and mortar. After obtaining a homogeneous granulate, residual IPA was removed under reduced pressure until the IPA content was less than 0.1 % w/w. The dried granulate was milled in a coffee grinder until the average particle size was not more than 100 µm (D [4,3]) and vacuum sealed in an alu-alu bag.

### Starch Granulate 2:

1.20 g of NHS-POx / Starch powder, 1.45 g of the NU-POx / Starch / Sodium Carbonate powder 3 and 0.15 mL of IPA were mixed using a pestle and mortar. After a homogeneous granulate was formed, residual IPA was removed under reduced pressure until the IPA content was less than 0.1 % w/w. The dried granulate was milled in a coffee grinder until the average particle size was not more than 100 µm (D [4,3]) and vacuum sealed in an alu-alu bag

### Preparation of reactive NHS-POx / Gelita Spon granules

7.01 g of pre-dried gelatin powder (Gelita-SPONO, ex Gelita Medical GmbH), having a water content of less than 0.2% w/w, was dispersed in dichloromethane (200 mL) using a high shear mixer operating at 20,000 rpm for 20 minutes. Subsequently, NHS-POx (7.02 g) was added and the stirring was continued for 5 minutes. NHS-POx did not dissolve. All volatiles were removed from the suspension under reduced pressure. The obtained powder was milled using a coffee grinder until the average particle size was not more than 95 µm (D [4,3]) and vacuum sealed in alu-alu bags, further dried under reduced pressure and vacuum sealed in an alu-alu bag.

The particle size distribution of the granulates so obtained was approximately: 90 vol.% < 190 µm, 50 vol.% < 80 µm and 10 vol.% < 15 µm.

The granulate was analysed by means of ¹H-NMR spectroscopy analysis. To this end deuterated chloroform (CDCl₃) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 97 percent compared to NHS-POx.

The granulate was further analysed by means of size exclusion chromatography (SEC) analysis. An aliquot (0.15 mL) of the filtered NHS-POx extract described above was diluted with *N,N*-dimethylacetamide containing 50 mM lithium chloride (1.00 mL), which was the eluent for SEC analysis. The sample was analysed by SEC against poly(methyl methacrylate) standards and the PDI was 1.45 indicating that no cross linking had occurred.

### Preparation of reactive NHS-POx / Chitosan granules

NHS-POx/Chitosan reactive granules were prepared as follows: 5 g of NHS-POx powder were wetted with IPA in a high shear mixer until a homogeneous snow like powder was obtained containing about 1-2% w/w IPA. After this, 5 g of Chitosan powder (Shanghai Waseta International, 85% DAC degree) were added and mixed. After mixing, the wet granulates were dried under reduced pressure until the IPA content was less than 0.1% w/w as determined via ¹H-NMR. The dried granulates were milled in a coffee grinder until the average particle size was not more than 200 µm (D [4,3]) and vacuum sealed in alu-alu bags. The particle size distribution of the granulates so obtained was approximately: 90 vol.% < 350 µm, 50 vol.% < 180 µm and 10 vol.% < 60 µm.

The granulate was analysed by means of ¹H-NMR spectroscopy analysis. To this end deuterated chloroform (CDCl₃) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 95 percent compared to NHS-POx.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 103 percent, indicating that no insoluble crosslinked material was formed.

The NHS-POX/Chitosan granulate was further analysed by means of size exclusion chromatography. Therefore, an aliquot (0.15 mL) was taken from the solution used for ¹H-NMR spectroscopy analysis. This solution was diluted with *N,N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking had occurred during granulation.

### Preparation of NHS-POx / Chitosan mixtures by co-freeze drying

Co-freeze dried NHS-POx/Chitosan powders were prepared as follows: 2.5 g of Chitosan powder (Shanghai Waseta International, 85% DAC degree) were dissolved in 200 mL of a 0.2% v/v acetic acid solution in ultrapure water. The pH was adjusted to 4.5 by the addition of acetic acid and the solution was cooled to 4°C. Subsequently, 2.5 g of NHS-POx was added and dissolved with the aid of high shear stirring. Directly after complete dissolution of the NHS-POx, the solution was flash frozen in liquid nitrogen and freeze dried. The resulting powder was dried under reduced pressure and vacuum sealed in an alu-alu bag.

The granulate was analysed by means of ¹H-NMR spectroscopy analysis. To this end deuterated chloroform (CDCl₃) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 12 percent compared to NHS-POx, which implies that crosslinking and/or hydrolysis occurred.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 11 percent (in line with the NMR results), indicating that insoluble crosslinked material was formed.

The NHS-POX/Chitosan granulate was further analysed by means of size exclusion chromatography. Therefore, an aliquot (0.15 mL) was taken from the solution used for ¹H-NMR spectroscopy analysis. This solution was diluted with *N,N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was determined to be 5.4, indicating that crosslinking took place.

### Preparation of NHS-POx / Chitosan mixtures by dry mixing

NHS-POx/Chitosan mixtures were prepared as follows: 2 g of Chitosan powder (Shanghai Waseta International, 85% DAC degree) and 2 g of NHS-POx were dry mixed by means of tumble mixing for 30 minutes. The resulting powder was dried under reduced pressure and vacuum sealed in an alu-alu bag.

The powder was analysed by means of ¹H-NMR spectroscopy analysis. To this end deuterated chloroform (CDCl₃) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 99 percent compared to NHS-POx.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 96 percent, indicating that no insoluble crosslinked material was formed.

The NHS-POX/Chitosan powder was further analysed by means of size exclusion chromatography. Therefore, an aliquot (0.15 mL) was taken from the solution used for ¹H-NMR spectroscopy analysis. This solution was diluted with *N,N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking had occurred during dry mixing.

### Cohesive fibrous carrier structures

The following commercially available haemostatic product was selected to be used as fibrous carrier structures in the preparation of tissue-adhesive sheets (not according to the claimed invention):
- Gelita Tuft-It^{®}: A cohesive fibrous carrier structure consisting of eight layers of reduced cross-linked gelfoam fibres. The eight layers, of each about 2 mm thickness, have dimensions of 50 mm by 75 mm. The water content of Gelita Tuft-It^{®} is not more than 15%. The product was dried in a vacuum oven for several hours at 40°C to reduce the water content to not more than 2.0% w/w (determined gravimetrically), before it was impregnated with agglomerate particles.

### Bleeding experiments

Standardized *ex-vivo* and *in-vivo* porcine bleeding models were used to assess haemostatic efficacy. All models use heparin to increase clotting time of blood to about 2 to 3 times activated coagulation time (ACT).

*Ex-vivo* model: live *ex-vivo* pig model with a fresh liver, perfused with heparinized fresh blood from the slaughterhouse to mimic real *in-vivo* situations a closely as possible. Livers are mounted onto a perfusion machine by which oxygenation, pH of blood, temperature and blood pressure are kept within *vivo* boundaries. Two livers and 10 litres of heparinized blood (5000 units/L) are collected at the slaughterhouse. Livers are transported on ice; blood at ambient temperature. Within two hours after collection, livers are inspected for lesions which are closed with gloves and cyanoacrylate glue.
- Perfusion parameters: flow 600 ml/min; pressure 10-12 mmHg; temperature 37 °C (+/- 1 °C); carbogen 0.25 litres a minute
- With a flat, round, rotating abrasion tool a circular bleeding wound (8 mm diameter) is created on the liver surface, with a rubber onlay so that the depth of the punched bleeding is always 3 mm
- After the liver is perfused properly (colour and temperature checked) samples are tested according to the following procedure: cut sample to the right size (2.7 by 2.7 cm); camera on; site number on camera; biopsy punch 8 mm; cut away biopt; remove blood from bleeding with gauze (2x); collect blood for 30 sec in pre-weight gauze; score bleeding (by 2 researchers); pour haemostatic powder on bleeding site and use stainless steel spatula to evenly distribute powder; observe for 5 min (check and score adhesion and coagulation) and repeat after 30 minutes.

*In-vivo* model: standardized combined penetrating spleen rupture is inflicted in anesthetized swine (Domestic Pig, Male, Body Weight Range: 40 kg, Adult). A midline laparotomy is performed to access the spleen and other organs. Using a scalpel, n=3 (S1 ... S3) subcapsular standardized lesions (10 mm x 10 mm) are made. The haemostatic products are applied with gentle pressure by a pre-wetted gauze (saline) and held for 1 min. After application of the product the time to haemostasis (TTH) is assessed. If TTH equals zero, this means that after 1 minute pressure haemostasis had already been achieved.

### Scoring system for granulates: Coagulation

| | |
|---|---|
| ++++ | Achieved immediately after application |
| +++ | Achieved <10 seconds after application |
| ++ | Achieved <30 seconds after application |
| + | Achieved within 3 minutes after application |
| - | Not achieved |

### Scoring system for granulates: Adhesion 10 minutes after application

| | |
|---|---|
| ++++ | Very strong adhesion (coagulated granulates can hardly be removed) |
| +++ | Strong adhesion (coagulated granulates difficult to be removed) |
| ++ | Strong adhesion (coagulated granulates can be removed) |
| + | Moderate adhesion (coagulated granulates easily to be removed) |
| +/- | Mild adhesion (coagulated granulates removed by blood flow) |
| - | Not achieved |

### Scoring system for patches: Coagulation

| | |
|---|---|
| ++++ | Achieved immediately after tamponade |
| +++ | Achieved <10 seconds after tamponade |
| ++ | Achieved <30 seconds after tamponade |
| + | Achieved within 3 minutes after tamponade |
| +/- | Achieved after 3 minutes, second tamponade applied |
| - | Not achieved |

### Scoring system for patches: Adhesion 10 minutes after application

| | |
|---|---|
| ++++ | Very strong adhesion (patch breaks when being removed) |
| +++ | Strong adhesion (patch breaks when being removed) |
| ++ | Strong adhesion (patch can be removed without breaking) |
| + | Moderate adhesion (patch can be removed without breaking) |
| +/- | Mild adhesion (patch can be removed without breaking) |
| - | Not achieved |

### Example 1

The haemostatic properties of the different reactive granulates was evaluated in the ex *vivo* and *in vivo* bleeding tests described above. The results are summarised in Table 1 and table 2.

**Table 1**

| **Granulate** | ***Ex vivo*** | | ***In vivo*** | |
|---|---|---|---|---|
| | **Coagulation** | **Adhesion** | **Coagulation** | **Adhesion** |
| NHS-POx/NU-POx | +++ | +++ | +++ | +++ |
| NHS-POx/NU-POx/P188 2.5% | +++ | +++ | n.a. | n.a. |
| NHS-POx/Gelita Spon | ++++ | ++ | ++++ | ++ |
| NHS-POx/RXL-HS | n.a. | n.a. | + | ++++ |
| NHS-POx/Chitosan | +++ | ++ | n.a. | n.a. |
| NHS-POx/RXL-LS | n.a. | n.a. | + | ++++ |
| NHS-POx/RXL-HS carbonate | n.a. | n.a. | + | ++++ |
| NHS-POx/RXL-LS carbonate | n.a. | n.a. | + | ++++ |
| NHS-POx / NH2-PEG | n.a. | n.a. | ++ | ++ |
| NHS-POx / NU-POx, sequestered | +++ | ++ | n.a. | n.a. |
| Starch Granulate-1 | n.a. | n.a. | +++ | ++ |
| Starch Granulate-2 | n.a. | n.a. | ++++ | ++ |

**Table 2**

| **Granulate / Powder** | **Non-reacted NHS (%)** | **Recovery (%)** | **PDI** | ***Ex vivo*** | |
|---|---|---|---|---|---|
| | | | | **Coagulation** | **Adhesion** |
| NHS-POx / NU-POx granulate | 98 | 99 | < 1.5 | +++ | +++ |
| NHS-POx / NU-POx dry mixed | 99 | 101 | < 1.5 | + | + |
| NHS-POx / NU-POx co-freeze dried | n.d.^{[1]} | n.d.^{[1]} | n.d.^{[1]} | n.d. | n.d. |
| NHS-POx / RXL granulate | 98 | 105 | < 1.5 | ++ | +++ |
| NHS-POx / RXL | 100 | 104 | < 1.5 | + | +/- |
| dry mixed | | | | | |
| NHS-POx / RXL co-freeze dried | 93 | 81 | 3.3 | - | - |
| NHS-POx / Chitosan granulate | 93 | 103 | < 1.5 | +++ | +++ |
| NHS-POx / Chitosan dry mixed | 99 | 96 | < 1.5 | + | ++ |
| NHS-POx / Chitosan co-freeze dried | 12 | 11 | 5.4 | - | +/- |

| | | | | | |
|---|---|---|---|---|---|
| Note: ^{[1]} not determined (powder was not soluble due to high degree of crosslinking) | | | | | |

### Example 2

### Impregnation of carriers structure with the particle agglomerates

Using mechanical shaking, Gelita Tuft-It^{®} patches (50x75 mm, appr. 0.71 g) were impregnated with blue dyed NHS-POx/NU-POx (1:0.6) granulate. A paint shaking machine was used (VIBA PRO V of Collomix GmbH) to introduce the powder (appr. 0.75 g) into the patch. The array with the carrier structures holder was clamped in the machine. The array was vibrated vertically.

The impregnated samples were put on a PMMA plate and placed in an oven in which samples were subjected to different heat treatments. To evaluate powder fixation, samples were ticked twice on the white PMMA plate. If no blue powder was released, the outcome was regarded as fixated. The results are shown in Table 3.

**Table 3**

| **Temperature (°C)** | **Time (min)** | **Fixation** |
|---|---|---|
| 70 | 15 | no fixation |
| 70 | 30 | no fixation |
| 70 | 60 | no fixation |
| 70 | 300 | no fixation |
| 75 | 15 | semi fixation |
| 75 | 30 | semi fixation |
| 80 | 15 | fixation |
| 80 | 30 | fixation |
| 85 | 15 | fixation |

The NHS-POx/NU-POx granulate is hygroscopic. At ambient temperature and relative humidity (RH) lower than 40%, fibrous carrier structures can be impregnated within half an hour of exposure, reproducibly. However, if impregnation is performed at, for instance, RH 75% and 25 °C the granulate gets sticky within minutes, leading to non-reproducible, inhomogeneous impregnation characteristics.

### Example 3 ( not according to the claimed invention)

Hemostatic patches (Gelita Tuft-It^{®}; 50 x 75 mm, appr. 0.7 g) were impregnated with the reactive NHS-POx/NU-POx (1:0.6) granulate previously described. One gram of the granulate was distributed throughout the patches as described in Example 2. Next, the hemostatic patches were packed in alu-alu pouches containing 1 g of silica and vacuum sealed.

Patches were cut into 2 cm x 2 cm pieces and tested in triplicate in the ex *vivo* liver perfused model. Time to haemostasis (TTH) was 0 (after 1 minute pressure) and no re-bleeding was observed during the 30 minutes observation time. The patch was also found to have great flexibility and bending properties.

The patches were also evaluated in the *in vivo* porcine heparinized model. They were found to have very good coagulation and adhesive properties. Active bleedings were efficiently stopped in resections of various organs: spleen, liver and kidney. A summary of the results is shown in Table 3.

**Table 3**

| **Organ** | ***Ex-vivo*** | | ***In-vivo*** | |
|---|---|---|---|---|
| | **Coagulation** | **Adhesive properties** | **Coagulation** | **Adhesive properties** |
| Liver | ++++ | +++ | ++++ | +++ |
| Spleen | NA | NA | ++++ | +++ |
| Kidney | NA | NA | ++++ | +++ |

### Example 4

Hemostatic patches (Gelita Tuft-It^{®}; 50 x 75 mm, appr. 0.7 g) were impregnated with NHS-POx/NU-POx/P188 2.5%. One gram of the granulate was distributed throughout the patches as described in Example 2. Next, the hemostatic patches were packed in alu-alu pouches containing 1 g of silica and vacuum sealed.

The patches were evaluated in the *in vivo* porcine heparinized model. They were found to have very good coagulation and sufficient adhesive properties - the reduced adhesive properties enabled the patch to be removed in one piece as opposed to identical patches that did not include P188. Active bleedings were efficiently stopped in resections of various organs: spleen, liver and kidney. A summary of the results is shown in Table 4.

**Table 4**

| **Organ** | ***Ex-vivo*** | | ***In-vivo*** | |
|---|---|---|---|---|
| | **Coagulation** | **Adhesive properties** | **Coagulation** | **Adhesive properties** |
| Liver | n.a. | n.a. | ++++ | ++ |
| Spleen | n.a. | n.a. | ++++ | ++ |
| Kidney | n.a. | n.a. | ++++ | ++ |

### Example 5 (not according to the claimed invention)

Gelita Tuft-It^{®} (50 x 75 mm, appr. 0.7 g) was impregnated with different reactive polymer powders. The haemostatic properties of the patches so obtained were tested in the *ex-vivo* and *in-vivo* porcine bleeding models described herein before.

The fibrous carrier structures were impregnated with 1.4 g of powder using a pneumatic shaking device. The sheet of fibrous carrier was vibrated vertically. The engine of the long stroke type (NTK 25 AL L, ex Netter Vibration GmbH) was operated at 6 bar, 11 Hz and an amplitude of 30 mm. Four cycles of 15 seconds were used to disperse the powder into the sheet. The granulates were distributed through the complete thickness of the sheets. Also the distribution over the surface of the sheets was homogeneous.

Seven different reactive granulates were tested. These granulates contained NHS-POx in combination with a water-soluble nucleophilic polymer. The preparation of these granulates has been described herein before.

The granulates that were tested are listed below:
- NHS-POx / Gelita Spon
- NHS-POx / RXL (high salt)
- NHS-POx / RXL (high salt) containing carbonate
- NHS-POx / RXL (low salt)
- NHS-POx / NH2-PEG

The different combinations of fibrous carrier structure and reactive polymer powders that were tested are shown in Table 5.

**Table 5**

| **Patch** | **Electrophilic polymer (EP)** | **Nucleophilic polymer (NP)** | **Carbonate (C)** |
|---|---|---|---|
| 1 | NHS-POx | Gelita Spon | |
| 2 | NHS-POx | NH₂-PEG | |
| 3 | NHS-POx | RXL-HS | |

| **Patch** | **Electrophilic polymer (EP)** | **Nucleophilic polymer (NP)** | **Carbonate (C)** |
|---|---|---|---|
| 4 | NHS-POx | RXL-LS | |
| 5 | NHS-POx | RXL-HS | X |

The results obtained with these patches in the *ex-vivo* and *in-vivo* porcine bleeding models are summarised in Table 6.

**Table 6**

| **Patch** | ***Ex vivo*** | | ***In vivo*** | |
|---|---|---|---|---|
| | **Coagulation** | **Adhesion** | **Coagulation** | **Adhesion** |
| 1 | ++++ | ++ | ++++ | ++ |
| 2 | n.a. | n.a. | + | + |
| 3 | n.a. | n.a. | + | + |
| 4 | n.a. | n.a. | + | + |
| 5 | n.a. | n.a. | + | + |

### Comparative Example A

7.03 g of NHS-POx were dispersed in 50 mL dichloromethane (DCM) . A turbid mixture was formed and IPA (4 mL) was added slowly to obtain a clear solution; 7.00 g of pre-dried gelatin (Gelita-SPONO, Gelita Medical GmbH) were dispersed in DCM/IPA (150 mL/12 mL) using a high shear mixer operating at 20,000 rpm at room temperature for 20 minutes. The prepared NHS-POx solution was added and the dispersion was stirred at 20,000 rpm for 5 minutes. Subsequently, all volatiles were removed under reduced pressure. The formed granulate was milled using a coffee grinder, dried under reduced pressure and vacuum sealed in an alu-alu bag.

The granulate was analysed using ¹H-NMR spectroscopy and size exclusion chromatography. The results so obtained showed that no NHS-POx was present in the gelatin / NHS-POx granulate, indicating that complete crosslinking between NHS-POx and gelatin had occurred.

### Example 6 (not according to the claimed invention)

Experiments were conducted to determine the effect of NU-POx content of the reactive NHS-POx/NU-POx granulate on *in-vivo* performance of the haemostatic patch.

### Method of impregnation

Hemostatic patches (Gelita Tuft-ItO; 50 x 75 mm, appr. 0.7 g) were impregnated with reactive NHS-POx / NU-POx granules made via acetone granulation in molar ratios of 1:0.10, 1:0.20 and 1:0.40, said molar ratio referring to the ratio of the number of NHS groups provided by NHS-POx to the number of amine groups provided by the NU-POx. The same hemostatic patches were also impregnated with reactive NHS-POx powder.

One gram of the granulate/powder was distributed throughout the patches using the Fibroline SL-Preg laboratory machine. Next, the hemostatic patches were fixated, dried and packed in alu-alu pouches containing 1 g of silica and vacuum sealed.

### Machine

The Fibroline SL-Preg laboratory machine moves particles between electrodes by applying voltages up to 40 kV at frequencies of up to 200 Hz for a period of up to 60 seconds. The two electrode plates have a size of about 50x40 cm. The top plate is grounded.

The following standard settings were used: 40 kV, 100 Hz, 20 seconds.

### Arrays

Powders were dosed gravimetrically into a 3D printed PMMA array after the array had been mounted onto the bottom electrode plate. The array was filled with reactive polymer powders using a scraping carton or metal spatula. The array measured 50 x 75 x 4 mm and contained 22x33 = 726 square wells (inner dimensions of each well: 2 x 2 x 2 mm). The combined volume of the 726 wells was approximately 5.8 mL.

### Spacer

A spacer mask was placed on top of the array. The spacer was used to allow particles to move up and down when subjected to the alternating electric field. If no spacer is used, penetration and distribution through the carrier is limited. For TUFT-IT this was a mask of 3 mm. This results in 3 + 4 mm = 7 mm distance of the electrodes.

The *in vivo* performance of haemostatic patches containing NHS-POx:NU-POx granulate (0, 10, 20 and 40 percent amine groups from NU-POx, the percentage being calculated on the basis of the number of NHS groups provided by the NHS-POx) or NHS-POx powder was evaluated in a-non heparinised *in-vivo* porcine model. The details of the patches that were tested are shown in Table 7.

**Table 7**

| **Patch** | **Molar % amine** | **NHS-POx : NU-POx (g/g)** | **Grams of granulate in patch** |
|---|---|---|---|
| 1 | 10 | 1:0.12 | 1 |
| 2 | 20 | 1 :0.25 | 1 |
| 3 | 40 | 1:0.48 | 1 |
| 4 | 0 | 1:0 | 1 |

### In vivo tests

Tests were carried out on adult female domestic pigs (40-50 kg) No anticoagulation agent was applied. Patch performance was tested on both spleen and liver. The spleen or liver were located and externalized as needed as the testing period progressed and their natural humidity was kept by covering them with saline soaked sponges.

Different types of injuries were created:
- Liver: Abrasions, biopsy punches and resections
- Spleen: Resections

An appropriately sized section of the liver parenchyma was abraded/punched to cause moderate to severe bleeding. The liver abrasions were created by surgical scalpel and a template of 1 × 1 cm2 and the circular punches using a 8 mm circular biopsy punch. Liver and spleen resections were created using a surgical knife.

The patch was applied immediately after the tissue resection or scarification:
- 2 x 2 cm pieces for the biopsy punches and abrasions
- Complete 7.5 x 5 cm patch for resections

The tested patches were applied on the bleeding tissue and gently pressed down by compression using a pre-wet gauze with saline solution. Tamponade was applied for an initial period of 10 seconds followed by subsequent 30 seconds intervals up to a total of 5 minutes.

A TUFT-IT patch that had not been impregnated was used as a reference (referred to as TUFT-IT).

The results of the *in vivo* tests are summarised in Table 8.

**Table 8**

| | **Average time to haemostasis (in seconds)** | | | |
|---|---|---|---|---|
| | **Liver abrasion** | **Liver punch** | **Liver resection** | **Spleen resection** |
| Patch 1 | 10 | 10 | 10 | 10 |
| Patch 2 | 10 | 10 | 10 | 10 |
| Patch 3 | 10 | 10 | 10 | 80 |
| Patch 4 | 10 | 80 | 75 | 165 |
| TUFT-IT | 135 | 165 | 210 | 225 |

Patches 1 to 4 showed very strong tissue adhesion, whereas only mild adhesion was observed for the TUFT-IT patch.

Patches 1 and 2 showed no more than very limited swelling after application. Patches 3 to 4 showed more, but still acceptable, swelling.

### Example 7 (not according to the claimed invention)

Hemostatic patches (Gelita Tuft-ItO; 50 x 75 mm, appr. 0.7 g) were impregnated with either a solution of NHS-POx, NHS-POx powder or NHS-POx / NU-POx granulate. The NHS-POx / NU-POx granulate used was made via acetone granulation in a molar ratio of 1:0.20 (see Example 8).

A spraying solution containing NHS-POx was prepared by dissolving NHS-POx in a 1:1 mixture of isopropyl alcohol and dichloromethane (200 g/L). The patches were impregnated with 5 mL of this spraying solution using a glass laboratory sprayer and pressurized air in a single spraying cycle. The total amount of NHS-POx delivered in this way was 1 gram per patch. After impregnation the patches were allowed to dry inside an oven at 40°C for 2 hours, following which they were stored in a desiccator for 2 days before being packing in alu-alu pouches containing 1 g of silica and vacuum sealing.

In addition, patches were impregnated with 1 gram of NHS-POx powder or 1 gram of the NHS-POx / NU-POx granulate using the procedure described in Example 8.

The performance of the patches so prepared was tested in triplicate in the ex *vivo* liver perfused model under mild (<20 mL/min) and severe bleeding (>50 mL/min) conditions. With a flat, round, rotating abrasion tool a circular bleeding wound (8 mm diameter) was created on the liver surface, with a rubber onlay so that the depth of the punched bleeding was always 3 mm. The results are shown in Table 9.

**Table 9**

| **Type of impregnation** | ***Ex-vivo*** | | | |
|---|---|---|---|---|
| | ***Mild bleeding*** | | ***Severe bleeding*** | |
| | **Hemostatic capacity** | **Adhesive properties** | **Hemostatic capacity** | **Adhesive properties** |
| NHS-POx / NU-POx granulate | ++++ | ++++ | ++++ | ++++ |
| NHS-POx powder | +++ | ++++ | + | ++++ |
| NHS-POx solution | - | +/- | not tested | not tested |

### Example 8

Haemostatic powders were prepared by mixing NHS-POx / NU-POx granulate with a haemostatic starch powder (Arista^{™} AH, ex BARD). The NHS-POx / NU-POx granulate used was made via acetone granulation in a molar ratio of 1:0.20 (see Example 8).

The NHS-POx / NU-POx granulate was mixed in a pestle and mortar with the starch powder in a 80/20 and a 90/10 w/w ratio.

The gel formation capacity of these powder blends, the pure starch powder and the pure NHS-POx / NU-POx granulate was evaluated as follows:
- Test tubes were charged with 20 mg of powder sample
- 250 µL of heparinized sheep blood was added and the mixture was agitated immediately for 10 seconds using a vortex mixer
- After 2 minutes, the test tubes were placed upside down in order to determine if a gel had been formed
- In case a gel had been formed, 1 mL of water was added to the gel and after 30 minutes it was determined whether the gel was still intact.

The results are shown in Table 10.

**Table 10**

| **Material** | **Gel (250µL blood)** | **Stability in water** |
|---|---|---|
| starch (20 mg) | No | n.d. |
| starch (200 mg) | Yes | No |
| NHS-POx/NU-POx (20 mg) | Yes | Yes |
| NHS-POx/NU-POx (2 mg) | Yes | Yes |
| starch + 10% NHS-POx/NU-POx (20 mg) | Yes | Yes |
| starch + 20% NHS-POx/NU-POx (20 mg) | Yes | Yes |

## Claims

1. A haemostatic powder comprising at least 10 wt.% of particle agglomerates, said particle agglomerates having a diameter in the range of 1-500 µm and comprising:
(a) electrophilic polyoxazoline particles containing electrophilic polyoxazoline carrying at least 3 reactive electrophilic groups that are capable of reacting with amine groups in blood under the formation of a covalent bond; and
(b) nucleophilic polymer particles containing a water-soluble nucleophilic polymer carrying at least 3 reactive nucleophilic groups that, in the presence of water, are capable of reacting with the reactive electrophilic groups of the electrophilic polyoxazoline under the formation of a covalent bond between the electrophilic polyoxazoline and the nucleophilic polymer.

2. Haemostatic powder according to claim 1, wherein the nucleophilic polymer is a selected from protein, chitosan, nucleophilic polyoxazoline, nucleophilic polyethylene glycol, polyethyleneimine and combinations thereof.

3. Haemostatic powder according to claim 2, wherein the nucleophilic polymer is selected from gelatin, collagen and combinations thereof.

4. Haemostatic powder according to claim 3, wherein the nucleophilic polymer is gelatin, preferably reduced crosslinked gelatin.

5. Haemostatic powder according to claim 2, wherein the nucleophilic polymer is nucleophilic polyoxazoline.

6. Haemostatic powder according to claim 2, wherein the nucleophilic polymer is chitosan.

7. Haemostatic powder according to any one of the preceding claims, wherein the electrophilic polyoxazoline contains at least 8 reactive electrophilic groups.

8. Haemostatic powder according to any one of the preceding claims, wherein the reactive electrophilic groups of the electrophilic polyoxazoline are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, olefins, glycidyl ethers, carboxyl, succinimidyl esters, sulfo succinimidyl esters, maleimido (maleimidyl), ethenesulfonyl, imido esters, aceto acetate, halo acetal, orthopyridyl disulfide, dihydroxy-phenyl derivatives, vinyl, acrylate, acrylamide, iodoacetamide and combinations thereof

9. Haemostatic powder according to any one of the preceding claims, wherein the particle agglomerates contain at least 10 wt.% of the electrophilic polyoxazoline and at least 10 wt.% of the nucleophilic polymer.

10. Haemostatic powder according to any one of the preceding claims, wherein the combination of the electrophilic polyoxazoline and the nucleophilic polymer constitutes at least 50 wt.% of the particle agglomerates.

11. Haemostatic powder according to any one of the preceding claims, wherein the ratio between the total number of reactive electrophilic groups provided by the electrophilic polyoxazoline and the total number of reactive nucleophilic groups provided by the nucleophilic polymer lies in the range of 1:1.5 to 1.5:1.

12. A method of preparing haemostatic particle agglomerates of:
(a) electrophilic polyoxazoline particles containing an electrophilic polyoxazoline carrying at least 3 reactive electrophilic groups that are capable of reacting with amine groups in blood under the formation of a covalent bond; and
(b) nucleophilic polymer particles containing a water-soluble nucleophilic polymer carrying at least 3 reactive nucleophilic groups that are capable of reacting with the reactive electrophilic groups of the electrophilic polyoxazoline under the formation of a covalent bond between the electrophilic polyoxazoline and the nucleophilic polymer;
said method comprising the step of combining 100 parts by weight of the electrophilic polyoxazoline particles with 10 to 1000 parts by weight of the nucleophilic polymer particles in the presence of non-aqueous granulation liquid.

13. Method according to claim 12, wherein the non-aqueous granulation liquid contains at least 60 wt.% of an organic solvent selected from isopropyl alcohol, ethanol, methanol, diethyl ether, heptane, hexane, pentane, cyclohexane, dichloromethane, acetone, and mixtures thereof.

14. Method according to claim 12 or 13, wherein the method comprises the step of wetting the electrophilic polyoxazoline particles with the non-aqueous granulation liquid, followed by the step of combining the wetted electrophilic polyoxazoline particles with the nucleophilic polymer particles.

## Patentansprüche

1. Hämostatisches Pulver, das mindestens 10 Gew.-% Teilchenagglomerate enthält, wobei die besagten Teilchenagglomerate einen Durchmesser im Bereich von 1-500 µm haben und Folgendes umfassen:
(a) elektrophile Polyoxazolinteilchen, die elektrophiles Polyoxazolin enthalten, das mindestens 3 reaktive elektrophile Gruppen trägt, die mit Amingruppen im Blut unter Bildung einer kovalenten Bindung reagieren können; und
(b) nukleophile Polymerteilchen, die ein wasserlösliches nukleophiles Polymer enthalten, das mindestens 3 reaktive nukleophile Gruppen trägt, die bei Vorhandensein von Wasser mit den reaktiven elektrophilen Gruppen des elektrophilen Polyoxazolins unter Bildung einer kovalenten Bindung zwischen dem elektrophilen Polyoxazolin und dem nukleophilen Polymer reagieren können.

2. Hämostatisches Pulver nach Anspruch 1, wobei das nukleophile Polymer aus Protein, Chitosan, nukleophilem Polyoxazolin, nukleophilem Polyethylenglykol, Polyethylenimin und Kombinationen davon ausgewählt ist.

3. Hämostatisches Pulver nach Anspruch 2, wobei das nukleophile Polymer aus Gelatine, Kollagen und Kombinationen davon ausgewählt ist.

4. Hämostatisches Pulver nach Anspruch 3, wobei das nukleophile Polymer Gelatine ist, vorzugsweise reduzierte vernetzte Gelatine.

5. Hämostatisches Pulver nach Anspruch 2, wobei das nukleophile Polymer nukleophiles Polyoxazolin ist.

6. Hämostatisches Pulver nach Anspruch 2, wobei das nukleophile Polymer Chitosan ist.

7. Hämostatisches Pulver nach einem der vorhergehenden Ansprüche, wobei das elektrophile Polyoxazolin mindestens 8 reaktive elektrophile Gruppen enthält.

8. Hämostatisches Pulver nach einem der vorhergehenden Ansprüche, wobei die reaktiven elektrophilen Gruppen des elektrophilen Polyoxazolins aus Carbonsäureestern, Sulfonatestern, Phosphonatestern, Pentafluorphenylestern, p-Nitrophenylestern, p-Nitrothiophenylestern, Säurehalogenidgruppen, Anhydriden, Ketonen, Aldehyden, Isocyanato, Thioisocyanato, Isocyano, Epoxiden, aktivierten Hydroxylgruppen, Olefinen, Glycidylethern, Karboxyl, Succinimidylestern, Sulfosuccinimidylestern, Maleimido (Maleimidyl), Ethensulfonyl, Imidoestern, Acetoacetat, Haloacetal, Orthopyridyldisulfid, Dihydroxyphenylderivaten, Vinyl, Acrylat, Acrylamid, Iodacetamid und Kombinationen davon ausgewählt ist.

9. Hämostatisches Pulver nach einem der vorhergehenden Ansprüche, wobei die Teilchenagglomerate mindestens 10 Gew.-% des elektrophilen Polyoxazolins und mindestens 10 Gew.-% des nukleophilen Polymers enthalten.

10. Hämostatisches Pulver nach einem der vorhergehenden Ansprüche, wobei die Kombination aus dem elektrophilen Polyoxazolin und dem nukleophilen Polymer mindestens 50 Gew.-% der Teilchenagglomerate ausmacht.

11. Hämostatisches Pulver nach einem der vorhergehenden Ansprüche, wobei das Verhältnis zwischen der Gesamtzahl der reaktiven elektrophilen Gruppen, die durch das elektrophile Polyoxazolin bereitgestellt werden, und der Gesamtzahl der reaktiven nukleophilen Gruppen, die durch das nukleophile Polymer bereitgestellt werden, im Bereich von 1:1,5 bis 1,5:1 liegt.

12. Verfahren zum Herstellen von hämostatischen Teilchenagglomeraten aus Folgendem:
(a) elektrophilen Polyoxazolinteilchen, die ein elektrophiles Polyoxazolin enthalten, das mindestens 3 reaktive elektrophile Gruppen trägt, die mit Amingruppen im Blut unter Bildung einer kovalenten Bindung reagieren können; und
(b) nukleophilen Polymerteilchen, die ein wasserlösliches nukleophiles Polymer enthalten, das mindestens 3 reaktive nukleophile Gruppen trägt, die mit den reaktiven elektrophilen Gruppen des elektrophilen Polyoxazolins unter Bildung einer kovalenten Bindung zwischen dem elektrophilen Polyoxazolin und dem nukleophilen Polymer reagieren können;
wobei das Verfahren den Schritt des Kombinierens von 100 Gewichtsteilen der elektrophilen Polyoxazolinteilchen mit 10 bis 1000 Gewichtsteilen der nukleophilen Polymerteilchen bei Vorhandensein einer nicht wässrigen Granulationsflüssigkeit umfasst.

13. Verfahren nach Anspruch 12, wobei die nicht wässrige Granulationsflüssigkeit mindestens 60 Gew.-% eines organischen Lösungsmittels enthält, das aus Isopropylalkohol, Ethanol, Methanol, Diethylether, Heptan, Hexan, Pentan, Cyclohexan, Dichlormethan, Aceton und Mischungen davon ausgewählt ist.

14. Verfahren nach Anspruch 12 oder 13, wobei das Verfahren den Schritt des Benetzens der elektrophilen Polyoxazolinteilchen mit der nicht wässrigen Granulationsflüssigkeit umfasst, gefolgt von dem Schritt des Kombinierens der benetzten elektrophilen Polyoxazolinteilchen mit den nukleophilen Polymerteilchen.

## Revendications

1. Poudre hémostatique comprenant au moins 10 % en poids d'agglomérats de particules, lesdits agglomérats de particules ayant un diamètre dans la plage de 1 à 500 µm et comprenant :
(a) des particules de polyoxazoline électrophile contenant de la polyoxazoline électrophile portant au moins 3 groupes électrophiles réactifs qui sont capables de réagir avec des groupes amine dans le sang sous la formation d'une liaison covalente ; et
(b) des particules de polymère nucléophile contenant un polymère nucléophile hydrosoluble portant au moins 3 groupes nucléophiles réactifs qui, en présence d'eau, sont capables de réagir avec les groupes électrophiles réactifs de la polyoxazoline électrophile sous la formation d'une liaison covalente entre la polyoxazoline électrophile et le polymère nucléophile.

2. Poudre hémostatique selon la revendication 1, où le polymère nucléophile est choisi parmi une protéine, le chitosane, la polyoxazoline nucléophile, le polyéthylèneglycol nucléophile, la polyéthylèneimine et leurs combinaisons.

3. Poudre hémostatique selon la revendication 2, où le polymère nucléophile est choisi parmi la gélatine, le collagène et leurs combinaisons.

4. Poudre hémostatique selon la revendication 3, où le polymère nucléophile est de la gélatine, de préférence de la gélatine réticulée réduite.

5. Poudre hémostatique selon la revendication 2, où le polymère nucléophile est la polyoxazoline nucléophile.

6. Poudre hémostatique selon la revendication 2, où le polymère nucléophile est le chitosane.

7. Poudre hémostatique selon l'une quelconque des revendications précédentes, où la polyoxazoline électrophile contient au moins 8 groupes électrophiles réactifs.

8. Poudre hémostatique selon l'une quelconque des revendications précédentes, où les groupes électrophiles réactifs de la polyoxazoline électrophile sont choisis parmi esters d'acide carboxylique, esters de sulfonate, esters de phosphonate, esters de pentafluorophényle, esters de p-nitrophényle, esters de p-nitrothiophényle, groupes halogénure d'acide, anhydrides, cétones, aldéhydes, isocyanato, thioisocyanato, isocyano, époxydes, groupes hydroxyle activés, oléfines, éthers de glycidyle, carboxyle, esters de succinimidyle, esters de sulfo succinimidyle, maléimido (maléimidyle), éthènesulfonyle, imidoesters, acétoacétate, haloacétal, disulfure d'orthopyridyle, dérivés de dihydroxy-phényle, vinyle, acrylate, acrylamide, iodoacétamide et leurs combinaisons.

9. Poudre hémostatique selon l'une quelconque des revendications précédentes, où les agglomérats de particules contiennent au moins 10 % en poids de la polyoxazoline électrophile et au moins 10 % en poids du polymère nucléophile.

10. Poudre hémostatique selon l'une quelconque des revendications précédentes, où la combinaison de la polyoxazoline électrophile et du polymère nucléophile constitue au moins 50 % en poids des agglomérats de particules.

11. Poudre hémostatique selon l'une quelconque des revendications précédentes, où le rapport entre le nombre total de groupes électrophiles réactifs fournis par la polyoxazoline électrophile et le nombre total de groupes nucléophiles réactifs fournis par le polymère nucléophile est compris dans la plage de 1:1,5 à 1,5:1.

12. Procédé de préparation d'agglomérats de particules hémostatiques de :
(a) particules de polyoxazoline électrophile contenant une polyoxazoline électrophile portant au moins 3 groupes électrophiles réactifs qui sont capables de réagir avec des groupes amine dans le sang sous la formation d'une liaison covalente ; et
(b) particules de polymère nucléophile contenant un polymère nucléophile hydrosoluble portant au moins 3 groupes nucléophiles réactifs qui sont capables de réagir avec les groupes électrophiles réactifs de la polyoxazoline électrophile sous la formation d'une liaison covalente entre la polyoxazoline électrophile et le polymère nucléophile ;
ledit procédé comprenant l'étape consistant à combiner 100 parties en poids des particules de polyoxazoline électrophile avec 10 à 1000 parties en poids des particules de polymère nucléophile en présence d'un liquide de granulation non aqueux.

13. Procédé selon la revendication 12, où le liquide de granulation non aqueux contient au moins 60 % en poids d'un solvant organique choisi parmi l'alcool isopropylique, l'éthanol, le méthanol, l'éther diéthylique, l'heptane, l'hexane, le pentane, le cyclohexane, le dichlorométhane, l'acétone et leurs mélanges.

14. Procédé selon la revendication 12 ou 13, où le procédé comprend l'étape de mouillage des particules de polyoxazoline électrophile avec le liquide de granulation non aqueux, suivie de l'étape de combinaison des particules de polyoxazoline électrophile mouillées avec les particules de polymère nucléophile.
